(19) Europäisches Patentamt
European Patent Office
Office européen des brevets

(11) EP 3 434 255 A1

(12) EUROPEAN PATENT APPLICATION

(43) Date of publication:
**30.01.2019 Bulletin 2019/05**

(51) Int Cl.:
***A61K 8/64*** *(2006.01)* ***A61Q 19/08*** *(2006.01)*

(21) Application number: **17306000.5**

(22) Date of filing: **26.07.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **L'OREAL**
**75008 Paris (FR)**

(72) Inventors:
- **CAZARES DELGADILLO, Jennyfer**
**94152 Chevilly La Rue (FR)**
- **BANGA, Ajay K.**
**Atlanta, Georgia 30341 (US)**

(74) Representative: **Nony**
**11 rue Saint-Georges**
**75009 Paris (FR)**

# (54) IONTOPHORESIS METHOD, COMPOSITION AND KIT FOR DELIVERING N-ACETYL-3-TRIFLUOROMETHYL-PHENYL-(VALINE-GLYCINE) THROUGH THE SKIN

(57) A iontophoresis method of delivering a N-acylaminoamide inhibitor of elastase through the skin, the electrical method comprising: applying a selected current profile, in particular a selected positive current profile, either continuous direct current, pulsed current or a combination of both, from any device and/or support comprising at least one electrode to a biological subject, the continuous direct current, the pulsed current or the combination of both of a character and for a duration sufficient to transdermally deliver a N-acylaminoamide inhibitor of elastase to a biological subject, and transporting different rates of N-acylaminoamide inhibitor of elastase across the skin in accordance to the selected current mode.

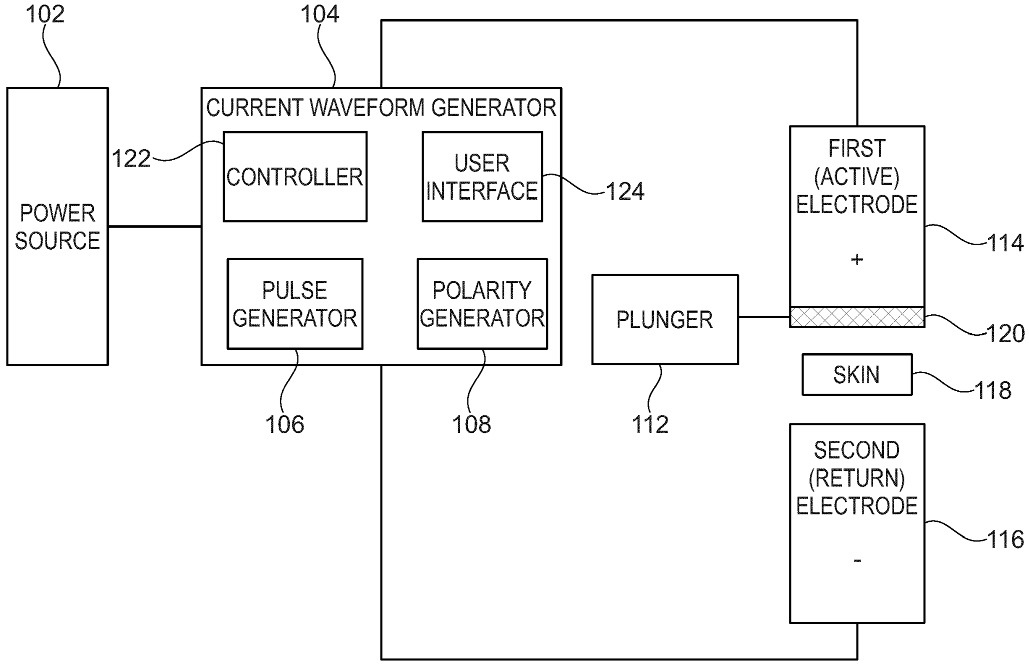


Fig. 1

EP 3 434 255 A1

**Description**

The present invention relates to the field of skincare and/or the care of skin appendages. The term "skin and/or its appendages" is intended to mean in particular the skin, the mucous membranes, the lips, the scalp, the eyelashes, the eyebrows and the hair.

A subject of the invention is a cosmetic treatment method for the skin and/or appendages thereof, comprising at least one step consisting in applying at least one composition comprising at least one N-acylaminoamide inhibitor of elastase, in particular one N-acetyl-3-trifluoromethyl-phenyl-(valine-glycine) or a derivative thereof to the skin. The method of the invention is other than therapeutic.

The present invention also relates to a topical cosmetic and/or dermatological composition comprising, in a physiologically acceptable medium, at least N-acetyl-3-trifluoromethyl-phenyl-(valine-glycine) or a derivative thereof.

It is known for example from EP 2 408 801 to use iontophoresis for delivering tetrapeptidic elastase inhibitors, and from WO2010/118880 to proceed to electrically enhanced transdermal application of tripeptides.

There is a need for improving the delivering of moderate-high size compounds, and in particular of dipeptides, such as N-acetyl-3-trifluoromethyl-phenyl-(valine-glycine), through the skin, without altering the skin.

Iontophoresis, a physical enhancement technique, that involves application of low current density (<0.5 mA/sq.cm), is used for enhancing the skin penetration of water soluble drug molecules, charged or neutral, that have low passive skin permeation.

As N-acetyl-3-trifluoromethyl-phenyl-(valine-glycine) is negatively charged at pH greater than 3.3, it has been explored the possibility of enhancing the permeability of that compound through keratinous material, and in particular through the skin, thanks to a negative electrical current (also called cathodal iontophoresis).

However, it has been found that a positive electrical current (also called anodal iontophoresis) is very effective for transporting different rates of N-acetyl-3-trifluoromethyl-phenyl-(valine-glycine) across the skin in accordance to the iontophoresis mechanism.

Electroosmosis is the principal transport mechanism of uncharged molecules and of high molecular weight cations (Santi, P., and R. H. Guy. 1996a. Reverse iontophoresis-parameters determining electroosmotic flow: I. pH and ionic strength. J. Control. Release. 38:159-165). The skin is negatively charged at physiological pH, and acts therefore as a permselective membrane to cations. This preferential passage of counterions induces an electroosmotic solvent flow that carries neutral molecules in the anode-to-cathode direction. The volume flow JV (volume × time-1 × area-1) is proportional to the potential gradient established by the electrical current

$$J_V = L_{VE} \times \frac{-d\Phi}{dx}$$

where $L_{VE}$ is the electroosmotic flow coefficient describing the direction and the magnitude of the volume flow resulting from the driving force, -dΦ/dx. The electroosmotic flux contribution to the transport of a solute *s* present in the anodal compartment at molar concentration $c_s$ is then

$$J_{EO} = J_V \times c_s$$

Electroosmosis assists the transport of high molecular weight cations and retards the passage of anions at pH 7. It can be modified by altering the permselectivity of the membrane and by manipulation of the formulation in the electrode chambers, that is by changing the value of $L_{VE}$ (Santi, P., and R. H. Guy. 1996a. Reverse iontophoresis-parameters determining electroosmotic flow: I. pH and ionic strength. J. Control. Release. 38:159-165).

**Electrical method**

In an embodiment of the invention, a method of delivering N-acylaminoamide inhibitor of elastase composition, in particular N-acetyl-3-trifluoromethyl-phenyl-(valine-glycine), for example an aqueous N-acetyl-3-trifluoromethyl-phenyl-(valine-glycine) composition, through the skin, comprises applying a selected current profile, either continuous direct current, pulsed current or a combination of both, from any device and/or support comprising at least one electrode to a biological subject, the continuous direct current, the pulsed current or the combination of both of a character and for a duration sufficient to transdermally deliver N-acylaminoamide inhibitor of elastase, in particular N-acetyl-3-trifluoromethyl-phenyl-(valine-glycine), for example an aqueous composition, to a biological subject, and transporting different rates of N-acylaminoamide inhibitor of elastase, in particular of N-acetyl-3-trifluoromethyl-phenyl-(valine-glycine), across the skin in accordance to the selected current mode.

This method may allow changes in skin impedance and thus an increase permeability of the N-acylaminoamide inhibitor of elastase, in particular N-acetyl-3-trifluoromethyl-phenyl-(valine-glycine), through keratinous material, and in particular through the skin.

N-acetyl-3-trifluoromethyl-phenyl-(valine-glycine) is a small peptide derivative, and its diffusion through the skin thanks to its ionization properties may be increased by the application of the iontophoresis method of the invention.

Such increase in its diffusion may enable to optimize the amount of active agent needed for target treatments into different layers of the skin.

The method of the invention also enable to increase the deposition of small negatively charged molecules into the skin with higher efficiency, by electroosmosis.

The method of the invention is cosmetic and non-therapeutic.

In an embodiment, the selected current profile is positive (anodal iontophoresis).

In iontophoresis, the applied electrical potential repels the charged species into and through the skin and this phenomenon, referred to as electromigration, electrorepulsion, or the Nernst-Planck effect is one of the mechanisms for driving the charged molecules into skin. However, besides electrorepulsion, electroosmosis also contributes to the enhancement in drug flux through skin. Electroosmotic flow is explained as the flux of bulk fluid as a result of potential difference applied across the skin and always occurs in the direction of the flow of counter ions. As skin is negatively charged, the direction of the movement of cations determines the electroosmotic flow which is always from anode to cathode. As a result, the cathodal delivery of anions is obstructed whereas the anodal delivery of cations is facilitated by electroosmosis. Anodal iontophoresis may also facilitate the movement of negatively charged active due to electroosmosis, and hence, it was observed significantly improved delivery of active agent by using anodal iontophoresis.

In some embodiments of the iontophoresis method, applying a selected current profile to a biological subject includes generating a continuous direct current stimulus having an average current density ranging from 0.001 mA/cm$^2$ to 0.5 mA/cm$^2$, preferably from 0.005 mA/cm$^2$ to 0.4 mA/cm$^2$, preferably from 0.01 mA/cm$^2$ to 0.3 mA/cm$^2$, and more preferably from 0.05 mA/cm$^2$ to 0.2 mA/cm$^2$, and in particular of 0.2 mA/cm$^2$.

In an embodiment, the continuous direct current stimulus has an average current density below 0.2 mA/cm$^2$.

In some embodiments of the iontophoresis method, applying a selected positive current profile to a biological subject includes generating a continuous positive direct current stimulus having an average current density ranging from 0.001 mA/cm$^2$ to 0.5 mA/cm$^2$, preferably from 0.005 mA/cm$^2$ to 0.4 mA/cm$^2$, preferably from 0.01 mA/cm$^2$ to 0.3 mA/cm$^2$, and more preferably from 0.05 mA/cm$^2$ to 0.2 mA/cm$^2$, and in particular of 0.2 mA/cm$^2$.

In an embodiment, the continuous positive direct current stimulus has an average current density below 0.2 mA/cm$^2$.

In some embodiments of the iontophoresis method, applying a selected current profile to a biological subject includes generating a continuous direct current stimulus having an average current density of 0.2 mA/cm$^2$. The selected current profile may be positive.

In some embodiments, the continuous direct current stimulus, in particular a continuous positive direct current stimulus, is applied for a duration ranging from 30 seconds to 120 minutes, preferably from 5 minutes to 50 minutes and more preferably from 10 minute to 40 minutes. In an embodiment, the duration of the continuous direct current stimulus, in particular of the continuous positive direct current stimulus, may be of 20 minutes.

In some embodiments of the iontophoresis method, applying a selected current profile to a biological subject includes generating a pulsed current, in particular a positive pulsed current, having sinusoidal waveforms, non-sinusoidal waveforms, or combinations thereof.

In some embodiments of the iontophoresis method, applying a selected current profile to a biological subject includes generating a pulsed current, in particular a positive pulsed current, having periodic square waveforms, rectangular waveforms, saw tooth waveforms, spiked waveforms, trapezoidal waveforms, triangle waveforms, or combinations thereof.

In some embodiments of the iontophoresis method, applying a selected current profile to a biological subject includes concurrently delivering the continuous direct current and the pulsed current and generating a pulsed current stimulus having an average current density ranging from 0.005 mA/cm$^2$ to 0.5 mA/cm$^2$, in particular from 0.05 mA/cm$^2$ to 0.5 mA/cm$^2$; a pulse duration ranging from 100 microseconds to 500 microseconds, in particular from 200 microseconds to 300 microseconds; and a pulse frequency ranging from 1 Hertz to 500 Hertz, in particular from 100 Hertz to 300 Hertz.

## N-acylaminoamide inhibitor of elastase

The N-acylaminoamide inhibitor of elastase according to the present invention is a compound of formula (I):

(I)

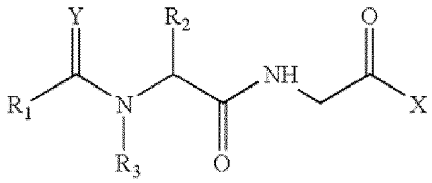

in which:

- the Y radical represents O or S;
- the $R_1$ radical represents:

  (i) a hydrogen atom;
  (ii) a hydrocarbon radical, linear, branched or cyclic, saturated or unsaturated, containing from 1 to 18 carbon atoms,
  optionally substituted by 1 to 5 groups, identical or different, selected from the group consisting of -OH; -OR; -O-COR; -SH; -SR; -S-COR; $-NH_2$; -NHR; -NRR'; -NH-COR; -Hal (halogen); -CN; -COOR; -COR; $-P(O)-(OR)_2$; and $-SO_2$-OR;
  with R and R' each representing independently a hydrocarbon radical, linear, branched or cyclic, saturated or unsaturated, containing 1 to 6 carbon atoms, optionally halogenated, said R and R' radicals being capable of forming together with N a 5- or 6- membered carbon ring that may include in addition at least one heteroatom selected from the group consisting of O, N and/or S in the ring, and/or may be substituted by 1 to 5 groups, identical or different, selected from the group consisting of -OH; -OR"; -O-COR"; -SH; -SR"; -S-COR"; $-NH_2$; -NHR"; -NH-COR"; -Hal (halogen); -CN; -COOR"; -COR"; $-C-Hal_3$ (halogen), in particular $-CF_3$ ; with R" representing independently a hydrocarbon radical, linear, branched or cyclic, saturated or unsaturated, containing 1 to 6 carbon atoms, optionally halogenated;
  (iii) a radical selected from the group consisting of the radicals -OR; $-NH_2$; -NHR; -NRR'; -NH-COR; -COOR; -COR;
  with R and R' each representing independently a hydrocarbon radical, linear, branched or cyclic, saturated or unsaturated, containing 1 to 6 carbon atoms, optionally halogenated; said R and R' radicals being capable of forming together with N a 5- or 6- membered carbon ring that may include in addition at least one heteroatom selected from the group consisting of O, N and/or S in the ring, and/or may be substituted by 1 to 5 groups, identical or different, selected from the group consisting of -OH; -OR"; -O-COR"; -SH; -SR"; -S-COR"; $-NH_2$; -NHR"; -NH-COR"; -Hal (halogen); -CN; -COOR"; and -COR"; with R" representing a hydrocarbon radical, linear, branched or cyclic, saturated or unsaturated, containing 1 to 6 carbon atoms, optionally halogenated,

- the radical $R_2$ represents a hydrocarbon radical, linear, branched or cyclic saturated or unsaturated, containing 1 to 18 carbon atoms, optionally substituted by 1 to 5 groups, identical or different, selected from the group consisting of -OH; -OR; -O-COR; -SH; -SR; -S-COR; $-NH_2$; -NHR; -NRR"; -NH-COR; -Hal (halogen); -CN; -COOR; and -COR; with R and R' each representing independently a hydrocarbon radical, linear, branched or cyclic, saturated or unsaturated, containing 1 to 6 carbon atoms, optionally halogenated, said R and R' radicals being capable of forming together with N a 5- or 6- membered carbon ring that may include in addition at least one heteroatom selected from the group consisting of O, N and/or S in the ring, and/or may be substituted by 1 to 5 groups, identical or different, selected from the group consisting of -OH; -OR"; -O-COR"; -SH; -SR"; -S-COR"; $-NH_2$; -NHR"; -NH-COR"; -Hal (halogen); -CN; -COOR"; and -COR"; with R" representing a hydrocarbon radical, linear, branched or cyclic, saturated or unsaturated, containing 1 to 6 carbon atoms, optionally halogenated,
- the radical $R_3$ represents a radical selected from the group consisting of those of formula (II) or (III) :

$$-A-C_6H_{(5-y)}-B_y \qquad (II)$$

$$-C_6H_{(5-y)}-B_{y'} \qquad (III)$$

in which:

- y is an integer included between 0 and 5 ;
- y' is an integer included between 1 and 5;
- A is a divalent hydrocarbon radical, linear, branched or cyclic, saturated or unsaturated, containing 1 to 18

carbon atoms, optionally substituted by 1 to 5 groups, identical or different, selected from the group consisting of -OH; -OR; -O-COR; -SH; -SR; -S-COR; $-NH_2$; -NHR; -NRR'; -NH-COR; -Hal (halogen); -CN; -COOR; -COR; $-NO_2$; and $-SO_2$-OR;
with R and R' each representing independently a hydrocarbon radical, linear, branched or cyclic, saturated or unsaturated, containing 1 to 6 carbon atoms, optionally halogenated, said R and R' radicals being capable of forming together with N a 5- or 6- membered carbon ring that may include in addition at least one heteroatom selected from the group consisting of O, N and/or S in the ring, and/or may be substituted by 1 to 5 groups, identical or different, selected from the group consisting of -OH; -OR"; -O-COR"; -SH; -SR"; -S-COR"; $-NH_2$; -NHR"; -NH-COR"; -Hal (halogen); -CN; -COOR"; and -COR"; with R" representing a hydrocarbon radical, linear, branched or cyclic, saturated or unsaturated, containing 1 to 6 carbon atoms, optionally halogenated ;

- B is a hydrocarbon radical, linear, branched or cyclic, saturated or unsaturated, containing 1 to 18 carbon atoms, optionally substituted by 1 to 5 groups, identical or different, selected from the group consisting of -OH; -OR; -O-COR; -SH; -SR; -S-COR; $-NH_2$; -NHR; -NRR'; -NH-COR; -Hal (halogen); -C-$Hal_3$ (halogen), in particular $-CF_3$; -CN; -COOR; -COR; $-NO_2$; and $-SO_2$-OR;
with R and R' each representing independently a hydrocarbon radical, linear, branched or cyclic, saturated or unsaturated; containing 1 to 6 carbon atoms, optionally halogenated, said R and R' radicals being capable of forming together with N a 5- or 6- membered carbon ring that may include in addition at least one heteroatom selected from the group consisting of O, N and/or S in the ring, and/or may be substituted by 1 to 5 groups, identical or different, selected from the group consisting of -OH; -OR"; -O-COR"; -SH; -SR"; -S-COR"; $-NH_2$; -NHR"; -NH-COR"; -Hal (halogen); -CN; -COOR"; and -COR"; with R" representing a hydrocarbon radical, linear, branched or cyclic, saturated or unsaturated, containing 1 to 6 carbon atoms, optionally halogenated ;

- the radical X represents a radical selected from the group consisting of -OH; $-OR_4$, $-NH_2$, $-NHR_4$, $-NR_4R_5$, $-SR_4$, $-COOR_4$; and $-COR_4$;

with $R_4$ and $R_5$ each representing independently a hydrocarbon radical, linear, branched or cyclic, saturated or unsaturated, containing 1 to 6 carbon atoms, optionally substituted by 1 to 5 groups, identical or different, selected from the group consisting of -OH; -OR; -O-COR; -SH; -SR; -S-COR; $-NH_2$; -NHR; -NRR'; -NH-COR; -Hal (halogen); -CN; -COOR; and -COR;
with R and R' each representing independently a hydrocarbon radical, linear, branched or cyclic, saturated or unsaturated, containing 1 to 6 carbon atoms, optionally halogenated, said R and R' radicals being capable of forming together with N a 5- or 6- membered carbon ring that may include in addition at least one heteroatom selected from the group consisting of O, N and/or S in the ring, and/or may be substituted by 1 to 5 groups, identical or different, selected from the group consisting of -OH; -OR"; -O-COR"; -SH; -SR"; -S-COR"; $-NH_2$; -NHR"; -NH-COR"; -Hal (halogen); -CN; -COOR"; and -COR"; with R" representing a hydrocarbon radical, linear, branched or cyclic, saturated or unsaturated, containing 1 to 6 carbon atoms, optionally halogenated,
said $R_4$ and $R_5$ radicals being capable of forming together with N a 5- or 6 membered carbon ring that may include in addition at least one heteroatom selected from the group consisting of O, N and/or S in the ring, and/or may be substituted by 1 to 5 groups, identical or different, selected from the group consisting of -OH; -OR"; -O-COR"; -SH; -SR"; -S-COR"; $-NH_2$; -NHR"; -NH-COR"; -Hal (halogen); -CN; -COOR"; -COR"; with R" representing a hydrocarbon radical, linear, branched or cyclic, saturated or unsaturated, containing 1 to 6 carbon atoms, optionally halogenated,
its mineral or organic acid salts, its optical isomers, in isolated form or as a racemic mixture.

Preferably, the Y radical represents O and/or the X radical represents -OH.

According to a preferred embodiment, the Hal (halogen) is fluorine.

By hydrocarbon radical, linear, cyclic or branched, is meant in particular the radicals of the alkyl, aryl, aralkyl, alkylaryl, alkenyl and alkynyl type.

The group $C_6H_5$ present in the radical $R_3$ must be understood as a cyclic aromatic group.

Preferably, the radical Y represents oxygen.

Preferably, the radical $R_1$ represents hydrogen or a hydrocarbon radical, linear or branched, saturated or unsaturated, containing 1 to 12, and in particular 1, 2, 3, 4, 5 or 6 carbon atoms, optionally substituted.

In particular, the substituents may be selected from -OH, -OR and/or -P(O)-$(OR)_2$ with R representing a hydrocarbon radical, linear, branched or cyclic, saturated or unsaturated, containing 1 to 6 carbon atoms, optionally halogenated, even perhalogenated.

Preferably, the radical $R_1$ represents a methyl, ethyl, propyl or isopropyl radical, optionally substituted by a -OH or -P(O)-$(OR)_2$ group with R representing methyl, ethyl, propyl or isopropyl.

Preferably, the radical $R_2$ represents a hydrocarbon radical, linear or branched, saturated or unsaturated, containing 1 to 12, and in particular 1, 2, 3, 4, 5 or 6 carbon atoms, optionally substituted.

In particular, the substituents may be selected from -OH and -OR with R representing a hydrocarbon radical,

linear, branched or cyclic, saturated or unsaturated, containing 1 to 6 carbon atoms, optionally halogenated, even perhalogenated.

Preferably, the radical $R_2$ represents a methyl, ethyl, propyl, isopropyl, n-butyl, tert.butyl or isobutyl radical.

Preferably, the radical $R_3$ represents a radical of formula $-C_6H_{(5-y')}-B_{y'}$, for which y'=1, 2 or 3; or a radical of formula $-A-C_6H_{(5-y)}-B_y$ for which y=0, 1 or 2. Preferably, A is a divalent hydrocarbon radical, linear or branched, saturated or unsaturated, containing 1 to 12 carbon atoms, optionally substituted. The substituents of A are preferably selected from -Hal (halogen, even perhalogen); -CN; -COOR; $-NO_2$; $-SO_2-OR$; with R representing a hydrocarbon radical, linear, branched or cyclic, saturated or unsaturated, containing 1 to 6 carbon atoms, optionally halogenated, even perhalogenated.

Preferably, the radical $R_3$ represents a group selected from one of the following formulae:

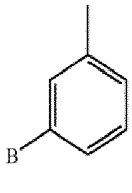
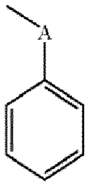
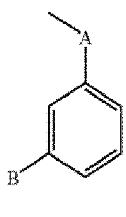
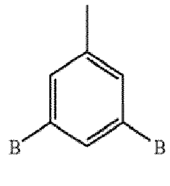

in which A and B have the above meanings.

In particular, the divalent radical A may be methylene, ethylene, propylene.

The radical B is preferably a methyl, ethyl, propyl or isopropyl radical, substituted by one or more halogens, in particular chlorine, bromine, iodine or fluorine, and preferably completely halogenated (perhalogenated), such as perfluorinated. Particular mention may be made of the perfluoromethyl radical ($-CF_3$) as very particularly preferred.

Preferably, the radical X represents a radical selected from -OH or $-OR_4$ with $R_4$ representing a hydrocarbon radical, linear, cyclic or branched, saturated or unsaturated, containing 1 to 6 carbon atoms, optionally substituted. The substituents may be selected from -OH and -OR with R representing a hydrocarbon radical, linear, branched or cyclic, saturated or unsaturated, containing 1 to 6 carbon atoms, optionally halogenated, even perhalogenated. Preferably, the radical X represents a radical selected from -OH, $-OCH_3$, $-OC_2H_5$, $-O-C_3H_7$ or $-OC_4H_9$.

The compounds according to the invention can be easily prepared by the specialist skilled in the art on the basis of his general knowledge. In particular, it is possible to react a carboxylic acid, an aldehyde, an amino compound and an isonitrile according to the Ugi reaction.

It is clearly understood that during the synthesis of the compounds according to the invention and as a function of the nature of the different radicals present in the starting compounds, the specialist will take care to protect certain substituents in order that they do not reaction in the course of the reactions.

The quantity of compound to be used in the compositions according to the invention can easily be determined by the specialist skilled in the art as a function of the nature of the compound used, the person to be treated and/or the effect desired. Generally, this quantity may be comprised between 0.00001% and 20% by weight of the total weight of the composition, preferably between 0.001% and 10% by weight.

The compounds of formula (I) may in particular be used in a composition which comprises a physiologically acceptable medium, in particular in a cosmetic or pharmaceutical composition which hence comprises moreover a cosmetically or pharmaceutically acceptable medium.

The physiologically acceptable medium in which the compounds according to the invention may be used as well as its constituents, their quantity, the formulation of the composition and its method of preparation can be chosen by the specialist skilled in the art on the basis of his general knowledge as a function of the type of composition desired.

Generally speaking, this medium can be anhydrous or aqueous. Thus it may comprise an aqueous phase and/or a fatty phase.

According to a preferred embodiment, a composition according to the invention may be in the form of an emulsion, and in particular in the form of a oil-in-water emulsion, also known as a direct emulsion.

According to a preferred embodiment, the composition according to the invention, especially when it is in the form of an emulsion, may also comprise at least one surfactant.

These surfactants may be chosen from nonionic, anionic, cationic and amphoteric surfactants, and mixtures thereof. Reference may be made to Kirk-Othmer's Encyclopedia of Chemical Technology, Volume 22, pp. 333-432, 3rd Edition, 1979, Wiley, for the definition of the emulsifying properties and functions of surfactants, in particular pp. 347-377 of this reference, for the anionic, amphoteric and nonionic surfactants.

According to a preferred embodiment, the composition may comprise at least one silicone surfactant.

The term "silicone surfactant" means a silicone compound that contains a hydrophilic chain and that is capable of emulsifying an aqueous phase in an oily phase. This silicone surfactant may be chosen from a group comprising

emulsifying silicone elastomers, dimethicone copolyols, alkyl dimethicone copolyols, and mixtures thereof.

According to a preferred embodiment, the silicone surfactant is chosen from the group of dimethicone copolyols and alkyl dimethicone copolyols.

When it is present in the composition, the content of surfactant(s) ranges from 0.1% to 7% by weight relative to the total weight of the composition.

**N- acetyl-3-trifluoromethyl-phenyl-(valine-glycine)**

In the context of the present invention, the term "N-acetyl-3-trifluoromethylphenyl-(valine-glycine)" covers in particular the basic unit of the following formula and its derivative:

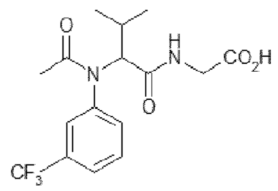

The term "N-acetyl-3-trifluoromethyl-phenyl-(valine-glycine) or a derivative thereof" also comprises, in the context of the present invention, the N-acetyl-3-trifluoromethyl-phenyl-(valine-glycine) salts, and in particular the alkali metals salts such as the sodium salt and the potassium salt.

N-acetyl-3-trifluoromethyl-phenyl-(valine-glycine) is an anti-aging agent.

In some embodiments of the electrical method, the method further comprises transdermally delivering a composition including N-acylaminoamide inhibitor of elastase, in particular one or more of N-acetyl-3-trifluoromethyl-phenyl-(valine-glycine), N-acetyl-3-trifluoromethyl-phenyl-(valine-glycine) derivatives, ions of N-acetyl-3-trifluoromethylphenyl-(valine-glycine), and ions of N-acetyl-3-trifluoromethyl-phenyl-(valine-glycine) derivatives, present in amounts ranging from 0.0001 % to 100 % by weight.

The N-acylaminoamide inhibitor of elastase, in particular the N-acetyl-3-trifluoromethyl-phenyl-(valine-glycine) or a derivative thereof, may be present in the composition according to the present invention at a content of between 0.001% and 20%, preferably between 0.01% and 10%, and more particularly between 0.01% and 5% by weight, relative to the total weight of the composition.

In an embodiment, the composition comprises 1% by weight of N-acylaminoamide inhibitor of elastase, and in particular of N-acetyl-3-trifluoromethylphenyl-(valine-glycine).

In an embodiment, the composition comprises 1% by weight of N-acylaminoamide inhibitor of elastase, and in particular of N-acetyl-3-trifluoromethylphenyl-(valine-glycine), ethanol and water. In an embodiment, the quantity of ethanol and water are equal in the composition.

In some embodiments of the iontophoresis method, the method further comprises transdermally delivering a composition, for example an aqueous composition, including N-acylaminoamide inhibitor of elastase, and in particular N one or more of N-acetyl-3-trifluoromethyl-phenyl-(valine-glycine), N-acetyl-3-trifluoromethyl-phenyl-(valine-glycine) derivatives, ions of N-acetyl-3-trifluoromethyl-phenyl-(valine-glycine), and ions of N-acetyl-3-trifluoromethyl-phenyl-(valine-glycine) derivatives, present in amounts ranging from 0.1 % to 20 % by weight; one or more silicon surface-active agents present in amounts ranging from 0.01 % to 30 % by weight; one or more ionic polymers present in amounts ranging from 0.01 % to 10% by weight; and water present in an amount of at least 30 % by weight.

In some embodiments of the iontophoresis method, the method further comprises:

transdermally delivering a composition including,

N-acylaminoamide inhibitor of elastase, and in particular N one or more of N-acetyl-3-trifluoromethyl-phenyl-(valine-glycine), N-acetyl-3-trifluoromethyl-phenyl-(valine-glycine) derivatives, ions of N-acetyl-3-trifluoromethyl-phenyl-(valine-glycine), and ions of N-acetyl-3-trifluoromethyl-phenyl-(valine-glycine) derivatives, present in amounts ranging from 0.01 % to 100 % by weight. preferably from 0.5 % to 60 % by weight.

In some embodiments of the iontophoresis method, the method further comprises

transdermally delivering an aqueous composition including,
one or more silicon surface-active agents present in amounts ranging from 0.01 % to 30 % by weight;
one or more ionic polymers present in amounts ranging from 0.01 % to 10 % by weight; and
water present in an amount of at least 30 % by weight.

In some embodiments of the iontophoresis method, the method further comprises transdermally delivering an aqueous composition including N-acylaminoamide inhibitor of elastase, and in particular one or more of N-acetyl-3-trifluoromethyl-phenyl-(valine-glycine), N-acetyl-3-trifluoromethyl-phenyl-(valine-glycine) derivatives, ions of N-acetyl-3-trifluoromethyl-phenyl-(valine-glycine), and ions of N-acetyl-3-trifluoromethylphenyl-(valine-glycine) derivatives, present in amounts ranging from 0.01 % to 30 % by weight; one or more silicon surface-active agents present in amounts ranging from 0.01 % to 30 % by weight; one or more non-ionic polymers present in amounts ranging from 0.01 % to 20 % by weight; and water present in an amount of at least 30 % by weight.

In some embodiments of the iontophoresis method, transdermally delivering N-acetyl-3-trifluoromethyl-phenyl-(valine-glycine), in particular the aqueous active agent composition includes, generating a continuous direct current stimulus having an average current density ranging from 0.01 mA/cm$^2$ to 0.5 mA/cm$^2$; and generating a pulsed current stimulus having an average current density ranging from 0.01 mA/cm$^2$ to 10 mA/cm$^2$; a pulse duration ranging from 10 microseconds to 500 microseconds; and a pulse frequency ranging from 10 Hertz to 500 Hertz; the continuous direct current and the pulsed current of a duration sufficient to transdermally deliver an aqueous active agent composition to a biological subject.

In some embodiments of the iontophoresis method, the method comprises a step of measuring at least one of the temperatures of the skin, the impedance of the skin, and a pH of the composition.

In some embodiments of the iontophoresis method, the application of current profile is reduced to a safety level when a measured value measured by one of the sensors exceeds a safety range or a safety value.

In some embodiments of the iontophoresis method, the method comprises a step of measuring the pH of the composition. When the measured pH exceeds a pH safety range, the application of current profile is switched to a safety level, for example a safety level less than 1V, such as 0,5V. The pH safety range may be pH 4 to 7. In some embodiment, when the measured pH exceeds a pH safety range, for example the range from 4 to 7, the device switches the polarity during a short time to enable to reequilibrate the pH.

In some embodiments of the iontophoresis method, the method comprises a step of measuring the impedance of the skin. When the measured impedance exceeds an impedance safety range, the application of current profile is reduced to a safety level to avoid adverse event. The safety level may be less than 1V, such as 0,5V. The impedance safety range may be 50 Ω to 1 MΩ.

In some embodiments of the iontophoresis method, the method comprises a step of measuring the temperature of the skin. When the measured temperature exceeds a temperature safety value, the application of current profile is switched to a safety level, for example less than 1V, such as 0,5V. The temperature safety value may be chosen less than 42°C.

In a preferred embodiments of the iontophoresis method, the method comprises the steps of:

- measuring the temperature of the skin, and
- measuring the impedance of the composition, and
- measuring the pH of the composition.

Then, the device is configured for treating the results and regulating the microcurrent and polarity.

**Composition**

In some embodiments, the invention also relates to an electrical composition comprising
N-acylaminoamide inhibitor of elastase, and in particular one or more of N-acetyl-3-trifluoromethyl-phenyl-(valine-glycine), N-acetyl-3-trifluoromethyl-phenyl-(valine-glycine) derivatives, ions of N-acetyl-3-trifluoromethyl-phenyl-(valine-glycine), and ions of N-acetyl-3-trifluoromethyl-phenyl-(valine-glycine) derivatives,
present in amounts ranging from 0.1 % to 30 % by weight, preferably in an amounts ranging from 0.5 % to 20 % by weight, 0.7 % to 10 % by weight, and
water present in an amount of at least 20 % by weight, preferably in an amount of at least 40 % by weight, more preferably in an amount of at least 45 % by weight.

The composition may also comprise ethanol present in an amount of at least 20 % by weight, preferably in an amount of at least 40 % by weight, more preferably in an amount of at least 45 % by weight.

The composition may comprise an organic buffer, such as HEPES, for example 10mM (pH 7.15) containing 25mM sodium chloride.

In some embodiments, an iontophoresis composition comprises one or more of N-acylaminoamide inhibitor of elastase, and in particular of N-acetyl-3-trifluoromethylphenyl-(valine-glycine), N-acetyl-3-trifluoromethyl-phenyl-(valine-glycine) derivatives, ions of N-acetyl-3-trifluoromethyl-phenyl-(valine-glycine), and ions of N-acetyl-3-trifluoromethyl-phenyl-(valine-glycine) derivatives present in amounts ranging from 0.1 % to 30 % by weight; one or more silicon materials, present in amounts ranging from 0.1 % to 30 % by weight; and water present in an amount of at least 20 % by weight; the iontophoresis composition having an aqueous phase that is at least 30% by weight relative to the total weight of the iontophoresis composition.

In some embodiments of the iontophoresis composition, the composition further comprises one or more ionic polymers present in amounts ranging from 0.01 % to 10% by weight; wherein the N-acylaminoamide inhibitor of elastase, and in particular the one or more of N-acetyl-3-trifluoromethyl-phenyl-(valine-glycine), N-acetyl-3-trifluoromethyl-phenyl-(valine-glycine) derivatives, ions of N-acetyl-3-trifluoromethylphenyl-(valine-glycine), and ions of N-acetyl-3-trifluoromethyl-phenyl-(valine-glycine) derivatives, are present in amounts ranging from 0.1 % to 30 % by weight.

In some embodiments of the iontophoresis composition, the composition further comprises one or more non-ionic polymers present in amounts ranging from 0.01 % to 20% by weight; wherein the N-acylaminoamide inhibitor of elastase , and in particular the one or more of N-acetyl-3-trifluoromethyl-phenyl-(valine-glycine), N-acetyl-3-trifluoromethyl-phenyl-(valine-glycine) derivatives, ions of N-acetyl-3-trifluoromethylphenyl-(valine-glycine), and ions of N-acetyl-3-trifluoromethyl-phenyl-(valine-glycine) derivatives are present in amounts ranging from 0.01 % to 30 % by weight.

In some embodiments of the iontophoresis composition, the composition further comprises a pH ranging from 2 to 7.5.

In some embodiment, when the measured pH exceeds a pH safety range, for example the range from 4 to 7, the device switches the polarity during a short time to enable to reequilibrate the pH.

## Iontophoresis kit

In some embodiments, the invention also provides an iontophoresis kit comprising:

- a iontophoresis composition including N-acylaminoamide inhibitor of elastase, and in particular one or more of N-acetyl-3-trifluoromethyl-phenyl-(valine-glycine), N-acetyl-3-trifluoromethyl-phenyl-(valine-glycine) derivatives, ions of N-acetyl-3-trifluoromethyl-phenyl-(valine-glycine), and ions of N-acetyl-3-trifluoromethyl-phenyl-(valine-glycine) derivatives, for example as described above, and
- an iontophoresis device for carrying the iontophoresis method as described above.

The invention also provides an iontophoresis kit comprising:

- a iontophoresis composition including N-acylaminoamide inhibitor of elastase, and in particular one or more of N-acetyl-3-trifluoromethyl-phenyl-(valine-glycine), N-acetyl-3-trifluoromethyl-phenyl-(valine-glycine) derivatives, ions of N-acetyl-3-trifluoromethyl-phenyl-(valine-glycine), and ions of N-acetyl-3-trifluoromethyl-phenyl-(valine-glycine) derivatives, for example as described above, and
- an iontophoresis device for delivering the iontophoresis composition through the skin, configured for: applying a selected positive current profile, either continuous direct current, pulsed current or a combination of both.

The continuous direct current, the pulsed current or the combination of both may be of a character and for a duration sufficient to transdermally deliver N-acylaminoamide inhibitor of elastase, and in particular N-acetyl-3-trifluoromethyl-phenyl-(valine-glycine), to a biological subject, and transporting different rates of N-acylaminoamide inhibitor of elastase, and in particular N-acetyl-3-trifluoromethyl-phenyl-(valine-glycine) across the skin in accordance to the selected current mode.

The selected positive current profile may be as described above in relation with the method.

The composition may be an aqueous composition.

The iontophoresis kit may be configured such that N-acylaminoamide inhibitor of elastase, and in particular N-acetyl-3-trifluoromethyl-phenyl-(valine-glycine), and water are already mixed in the composition when the composition is applied to the skin.

In some embodiments, the iontophoresis device may comprise at least one of a temperature sensor, an impedance sensor, and a pH sensor. The iontophoresis device may comprise at least two of a temperature sensor, an impedance sensor, and a pH sensor. In an embodiment, the iontophoresis device may comprise a temperature sensor, an impedance sensor, and a pH sensor.

The device may be configured such that the application of current profile is reduced to a safety level when a measured value measured by one of the sensors exceeds a safety range or a safety value.

In a preferred embodiment, the method, the composition and the kit above enable to treat wrinkles and ageing signs, to improve smoothness, quality of skin and appearance of the skin. It can thus be used in hydrating the skin, in improving the suppleness of the skin, in improving and/or decreasing the microrelief of the skin, and also in combating the signs of aging of the skin.

The e method, the composition and the kit above are in particular effective for delivery of anti-aging ingredients that permeate the skin beyond the superficial levels, which may contribute to such effects. After ablative laser procedures or chemical peels for instance, iontophoresis may also improve the healing process by achieving a higher delivery of actives and/or medications. Rapid penetration that achieves a greater depth while decreasing healing makes iontophoresis also good for acne scarring.

In another embodiment, the method is used to minimize skin anti-aging, and/or pigmentation, and/or volume, and/ or sagging wrinkle, and/or event tone and/or spots, and/or to improve firmness, and/or radiance, and/or smoothness, and/or softness of the skin. The method of the invention may be associated with the application of active agents associated to electrical current.

## DESCRIPTION OF THE DRAWINGS

The foregoing aspects and many of the attendant advantages of the disclosed subject matter will become more readily appreciated as the same become better understood by reference to the following detailed description, when taken in conjunction with the accompanying drawings, wherein:

FIGURE 1 is a schematic illustration of an iontophoresis device;
FIGURE 2 is a plot of an electrical waveform stimulus in accordance with one embodiment;
FIGURE 3 is a plot of an electrical waveform stimulus in accordance with one embodiment;
FIGURE 4 is a plot of an electrical waveform stimulus in accordance with one embodiment;
FIGURE 5 is a graph comparing delivery of the active from a cosmetic composition FLUIDANOV™ (passive diffusion versus cathodal iontophoresis) in receptor, stratum corneum and in skin over 20 min.
FIGURE 6 is a graph comparing delivery of the active from a simplex formulation (1% w/v active in water: ethanol (1:1) (passive diffusion versus cathodal iontophoresis) in receptor over 20 min.
FIGURE 7 is a graph comparing delivery of the active from a modified simplex formulation (1% w/v active in 10 mM PBS (pH 7.4): ethanol (1:1) (passive diffusion versus cathodal iontophoresis) in receptor, stratum corneum and in skin over 20 min.
FIGURE 8a is a graph comparing the permeation profile of the active from a new simplex formulation (1% w/v in HEPES buffer (pH 7.15) containing sodium chloride) over 4 hours (passive diffusion versus iontophoresis and anodal iontophoresis).
FIGURE 8b is a graph comparing delivery of active from a new simplex formulation (1% w/v in 10 mM HEPES buffer (pH 7.15) containing sodium chloride) (passive diffusion versus iontophoresis and anodal iontophoresis) according to one embodiment.
FIGURE 9 is a graph comparing delivery of the active from the modified simplex formulation (1% w/v active in PBS (pH 7.4): ethanol (1:1) (passive diffusion versus cathodal iontophoresis and anodal iontophoresis) in receptor, stratum corneum and in skin.
FIGURE 10 is a graph comparing delivery of the active from a cosmetic composition FLUIDANOT™ (passive diffusion versus cathodal iontophoresis and anodal iontophoresis) in receptor, stratum corneum and in skin.
FIGURE 11 is a graph comparing the amount of active delivered from a cosmetic composition (cathodal iontophoresis versus anodal iontophoresis) in receptor and in skin.

## DETAILED DESCRIPTION

N-acylaminoamide inhibitor of elastase, and in particular N-acetyl-3-trifluoromethyl-phenyl-(valine-glycine), and its various forms may have one or more beneficial uses. For example, N-acetyl-3-trifluoromethyl-phenyl-(valine-glycine) may act as an antiaging in humans. It may increase epidermal regeneration, and maintain epidermal homeostasis. With respect to lipids neosynthesis, it may increase ceramids, phospholipids, sphnigomyeline and cholesterol sulfate.

Iontophoresis is the process of conducting an electrical current for the purpose of transporting charged molecules into the skin. The technique involves the application of a mild electrical current to a charged molecule by using a similarly charged electrode as the molecule of interest to produce a repulsive effect that drives the charged molecules away from the electrode and into the skin.

In some embodiments, the effect of simple ionization (electromigration) is the main mechanism by which iontophoresis produces its transport properties. However, there are additional mechanisms called electroosmosis and electroporation that are produced by an electrical current. Electroosmosis induces a flow of solvent that carries uncharged molecules in the anode-to-cathode direction. In some embodiments, "iontophoresis" is the technique of using an electrical current to deliver molecules into the skin regardless of whether transport of the molecule is via electromigration or electroosmosis or electroporation.

Since many active molecules in skin care compositions have ionic forms, iontophoresis can be an effective tool

for the administration of these active molecules.

Referring to FIGURE 1, an iontophoresis device and electrode assembly is schematically illustrated. In some embodiments, the iontophoresis device and electrode assembly includes a power source 102, a current waveform generator 104, a first (active) electrode 114, a second (counter or return) electrode 116, a plunger 112, and a reservoir 120 at the end of the first electrode 114.

In an embodiment, the first (active) electrode 114 is the anode, which is connected to the positive pole of the generator, and the second (counter or return) electrode 116 is the cathode, which is connected to the negative pole of the generator.

It is to be appreciated that iontophoresis devices are available with additional features or fewer features. Therefore, other devices may include many other components that are not being shown or exclude some of the components that are shown. The purpose of FIGURE 1 is to illustrate and describe some of the major functional components of an iontophoresis device used to carry out one or more of the various embodiments of the methods for the application of N-acylaminoamide inhibitor of elastase, and in particular N-acetyl-3-trifluoromethyl-phenyl-(valine-glycine), compositions or other active agent compositions disclosed herein. It is to be appreciated that implicit and inherent in FIGURE 1 is the circuitry used to carry out the functions of the iontophoresis device. In some embodiments, the iontophoresis device is packaged as a hand-held device that is suitable for carrying in one hand during treatment. Treatment using hand-held devices includes constantly moving the iontophoresis device over the skin so that the active electrode 114 is moved across the surface of the skin while making contact. In some embodiments, the iontophoresis device is packaged as a stationary desk-top device, and the active electrode 114 is stationary and applied to a single location on the skin, such as through an adhesive. The major functional components of the iontophoresis device will now be described.

In some embodiments, the iontophoresis device includes a power source 102. A suitable power source would be any power source that can generate electrical current to power the various other circuits and devices. In some embodiments, a battery is used as the power source. Additionally, in some embodiments, an alternating power source coupled to a transformer can be connected to the power source 102. In some embodiments, the iontophoresis device is plugged into a wall socket. In some embodiments, the power source 102 produces continuous direct current. In some embodiments, circuitry is used to generate electrical waveforms other than continuous direct current.

The power source 102 has a negative pole and a positive pole. Generally, positive polarity will be applied to the first electrode 114. However, through circuitry and electrical devices, such as switches, the polarities of the first 114 and second 116 electrodes can be reversed momentarily to achieve pulse and alternating current waveforms.

In some embodiments, the power source 102 is connected to the current waveform generator 104. The current waveform generator 104 functions to generate various types of current waveforms. In some embodiments, the current waveform generator 104 generates the waveforms through hardware and software, such as circuitry, described herein. In some embodiments, the waveform generator 104 includes circuitry operably coupled to an electrode assembly, and the circuitry is configured to concurrently generate at least a continuous direct current stimulus and a pulsed current stimulus to the same electrode, the continuous direct current stimulus and the pulsed current stimulus of a character and for a duration sufficient to deliver a cosmetic composition to a biological subject. The current generator 104 is connected to the first 114 and the second 116 electrodes and is able to apply a potential across the electrodes to supply electrical current stimuli in various waveforms described herein. Toward that end, the current waveform generator 104 includes a pulse generator 106 and a polarity generator 108. In some embodiments, functionally, the pulse generator 106 generates pulses of current of controlled amplitude and duration. In some embodiments, functionally, the polarity generator 108 controls the polarity at the first electrode 114 and second electrode 116. In some embodiments, the polarity generator 108 maintains the polarities of the first 114 and second 116 electrodes constant. In some embodiments, the polarity generator 108 applies zero polarity to the first and second 116 electrodes. In some embodiments, the polarity generator 108 applies the polarity to the first and second 116 electrodes in pulses at a predefined rate and duration. In some embodiments, the polarity generator 108 reverses the polarities of the first 114 and second 116 electrodes at a predefined rate or interval. In some embodiments, the polarity generator 116 is configured to apply constant polarity, pulses, or reverse polarity for predefined durations, sequentially or in any order.

In some embodiments, the iontophoresis device will include a controller 122. In some embodiments, functionally, the controller 122 will receive input from the user interface 124 and, in conjunction with the pulse generator 106 and the polarity generator 108, control the current density waveforms at the specifications input by the user/operator. In some embodiments, the controller 122, pulse generator 106, and polarity generator 108 are implemented in hardware components, such as analog circuitry, digital circuitry, microprocessors, or combinations thereof, or software components.

In some embodiments, the controller 122 has instructions for guiding a user to input the various parameters depending on the waveform stimulus that is to be applied. The user interface 124 can prompt the user for the information. In some embodiments, the controller 122 will request the user to select the stimulus output from a constant (DC) value, a pulse wave, or both a constant value and a pulse wave for the current density stimulus output waveform. Once the controller 122 receives input of the one or more stimulus wave types, the controller 122 prompts the user on the user interface 124 for parameters corresponding to the selected wave output type or types.

In some embodiments, the iontophoresis device includes the user interface 124. In some embodiments, functionally, the user interface 124 is for entry of data relating to the waveform types to be applied as electrical stimuli. In some embodiments, the user interface 124 includes an alphanumeric keyboard and display. In some embodiments, the user interface 124 includes directional arrow buttons and an enter button to enter data into memory. In some embodiments, the alphanumeric keyboard is implemented as a touch screen display. In some embodiments, the input of wave parameters is through the use of text boxes. In some embodiments, the input of wave parameters is through the use of scrolling menus.

Regardless of the manner of data entry, in some embodiments, the user interface 124 communicates a variety of prompts for the user to input information. In some embodiments, the user interface 124 prompts the user to input the duration of the treatment step. In some embodiments, the duration of the treatment step is the sum of time of the application of an electrical current of any one or more wave types and includes the time when electrical current is off for pulse waves. For example, a pulse wave set with a duty cycle of 50% has the electrical current off during 50% of the time, meaning that each pulse is one-half of each pulse cycle. However, the treatment duration will include the off period of the pulse cycle.

In some embodiments, the user interface 124 provides options to allow the user to input whether the current density output will be continuous direct current, pulse current, alternating pulse current, or any combination, and the duration of each wave type. In some embodiments, the user interface 124 prompts the user whether different wave types are superimposed on each other. For example, a pulse wave can be superimposed on a continuous direct current wave. In some embodiments, the user interface 124 prompts the user whether different wave types are combined in sequence. In some embodiments, the user interface 124 prompts the user to input the current density values to output for each wave type. In some embodiments, the current density is input as average current density, root mean square current density, or peak current density. In some embodiments, the user interface 124 prompts the user to input the polarity at the first electrode 114, including positive, negative, or both for alternating, with the corresponding constant or pulsed current output. In some embodiments, the user interface 124 prompts the user to input the electrodes' cross-sectional areas. In some embodiments, the user interface 124 prompts the user to input skin temperature. In some embodiments, the user interface 124 prompts the user to input the frequency of pulses, the maximum and minimum amplitudes of pulses, and the duration of pulses. In some embodiments, the user interface 124 prompts the user to input the % duty cycle of unidirectional pulses. In some embodiments, the user interface 124 prompts the user to input the % duty cycle of respective bipolar pulses. In some embodiments, the user interface 124 prompts the user to specify the time between pulses. In some embodiments, the user interface 124 prompts the user to specify the duration of a wave packet (wave train), and the frequency of the wave packets. In some embodiments, the user interface 124 prompts the user to specify the number of pulses in a wave packet (wave train). In some embodiments, the user interface 124 prompts the user to input the pulse wave shape, including periodic square waveforms, rectangular waveforms, saw tooth waveforms, spiked waveforms, trapezoidal waveforms, or triangle waveforms. In some embodiments, the user interface 124 prompts the user to input the treatment area. In some embodiments, the user interface 124 prompts the user to specify whether to apply alternating negative and positive pulses. In some embodiments, the user interface 124 prompts the user to specify whether pulses are to be unipolar or bipolar.

A unipolar pulse means that pulsed electrical current travels in one direction. A bipolar pulse means that pulsed electrical current travels in two directions or reverses directions. In some embodiments, the user interface 124 prompts the user to specify a ratio of negative to positive pulses or a ratio of positive to negative pulses. In some embodiments, the user interface 124 prompts the user whether to combine any continuous direct current waveform with any pulse waveform to provide two or more different waveforms concurrently. In some embodiments, the user interface 124 prompts the user to apply two or more waveforms concurrently, synchronously, sequentially, or alternately. In some embodiments, the user interface 124 prompts the user to input the duration of each waveform and the cycle time of each waveform if the waveforms alternate. In some embodiments, the user interface 124 prompts the user for a pulse interval duration.

In some embodiments, when using two or more waveforms in a single treatment, the user interface 124 prompts the user to specify the treatment durations of the different waveforms, and how often each waveform cycles. In some embodiments when continuous direct current is used concurrently with a pulse wave, the user interface 124 prompts the user to specify the parameters of the pulse wave.

In some embodiments, the controller 122 carries out logic routines to direct the user interface 124 to present to the user the appropriate prompts so that the wave type information is entered. The controller 122 then uses the wave type parameters to generate, via the circuitry, including but not limited to the pulse generator 106 and the polarity generator 108, the appropriate stimulus wave according to the user entered parameters.

In some embodiments, the first electrode 114, also referred to as the active electrode, is connected to the power source 102 through the waveform generator 104. The first electrode 114 includes a reservoir 102 on the end thereof to hold a N-acylaminoamide inhibitor of elastase composition, and in particular a N-acetyl-3-trifluoromethyl-phenyl-(valine-glycine) composition or any active agent composition. In some embodiments, the reservoir 120 is a hollow indentation on the end of the first electrode 114, wherein the reservoir is used to contain a gel or gel-like N-acylaminoamide inhibitor

of elastase, and in particular N-acetyl-3-trifluoromethyl-phenyl-(valine-glycine), composition. Alternatively, in some embodiments, the reservoir 120 is an absorbent material to contain the N-acylaminoamide inhibitor of elastase, and in particular N-acetyl-3-trifluoromethyl-phenyl-(valine-glycine), composition. In some embodiments, generally, the design of the first electrode 114 contemplates the first electrode 114 having negative polarity. When the iontophoresis device is packaged as a hand-held device, the first electrode 114 is provided with a type of roll-on applicator, such as a ball and socket fed by the plunger 112.

The skin 118 represents the load on the system.

In some embodiments, the second electrode 116, also referred to as the counter or return electrode, is connected to the power source 102 through the waveform generator 104. The polarity of the second electrode 116 is maintained by the waveform generator 104 to be the opposite of the polarity of the first electrode 114.

In some embodiments, generally, the design of the second electrode 116 contemplates the second electrode 116 having negative polarity. In some embodiments, the second electrode 116 is a hand-held electrode that is held by the person receiving the treatment. In other embodiments, the second electrode 116 has an insulated cover and an exposed tip so that the second electrode is held by the device user and applied by the user on the skin of the person receiving the treatment.

In some embodiments, the reservoir 120 on the first electrode 114 is connected to a plunger 112. In some embodiments, functionally, the plunger 112 replenishes the N-acylaminoamide inhibitor of elastase, and in particular N-acetyl-3-trifluoromethyl-phenyl-(valine-glycine), composition or any active agent composition in the reservoir 120. In some embodiments, the plunger 112 includes a piston and push pod within a cylindrical container. In some embodiments, the plunger 112 can be operated by a trigger mechanism that is operated during treatment by the user of the iontophoresis device.

In some embodiments, an iontophoresis device includes active (donor) and return (counter) electrode assemblies, a skin contacting layer, and an active agent layer. In some embodiments, an iontophoresis device includes active and return electrode assemblies having a multi-laminate construction. In some embodiments, an iontophoresis device includes electrode assemblies with a multi-laminate construction configured to deliver an active agent composition through passive diffusion or iontophoresis.

In some embodiments, an iontophoresis device includes active and return electrode assemblies, each formed by multiple layers of polymeric matrices. In some embodiments, an iontophoresis device includes electrode assemblies having a conductive resin film electrode layer, a hydrophilic gel reservoir layer, aluminum or silver foil conductor layer, and an insulating backing layer.

In some embodiments, an iontophoresis device comprises an iontophoresis patch design. In some embodiments, an iontophoresis device comprises an iontophoresis multi-laminate design. In some embodiments, an iontophoresis device comprises an iontophoresis face mask design. In some embodiments, an iontophoresis device comprises an iontophoresis flexible substrate design.

In some embodiments, an electrode assembly includes at least one electrode and one or more compositions with an agent stored in a reservoir such as a cavity, a gel, a laminate, a membrane, a porous structure, a matrix, a substrate, or the like. Non-limiting examples of active agents include electrically neutral agents, molecules, or compounds capable of being delivered via electro-osmotic flow. In some embodiments, neutral agents are carried by the flow of, for example, a solvent during electrophoresis. In some embodiments, an iontophoresis device includes an electrode and a reservoir containing an effective amount of a N-acylaminoamide inhibitor of elastase, and in particular N-acetyl-3-trifluoromethyl-phenyl-(valine-glycine), composition or any agent composition.

In some embodiments, a reservoir includes any form or matter employed to retain an element, a composition, a compound, active agent, a pharmaceutical composition, and the like, in a liquid state, solid state, gaseous state, mixed state or transitional state. In some embodiments, a reservoir includes one or more ion exchange membranes, semipermeable membranes, porous membranes or gels capable of at least temporarily retaining an element, a composition, a compound, active agent, a pharmaceutical composition, electrolyte solution, and the like.

In some embodiments, a reservoir includes one or more cavities formed by a structure. In some embodiments, a reservoir serves to retain an element, a composition, a compound, active agent, a pharmaceutical composition, electrolyte solution, and the like prior to the discharge of such by electromotive force or current into a biological interface. In some embodiments, an electrode assembly includes one or more ion exchange membranes that may be positioned to serve as a polarity selective barrier between the active agent reservoir and a biological interface.

In some embodiments, an electrode assembly includes an electrode and a reservoir containing an effective amount of a N-acylaminoamide inhibitor of elastase, and in particular N-acetyl-3-trifluoromethyl-phenyl-(valine-glycine), composition or any aqueous active agent composition.

In some embodiments, the iontophoresis device includes circuitry that is coupled to the active electrode assembly, wherein the circuitry is configured to generate at least current stimuli from pulsed or continuous in a concurrent manner. In some embodiments, the circuitry is included in the iontophoresis device and the current waveform generator 104 illustrated in FIGURE 1. In some embodiments, circuitry applies a potential across the active and counter electrodes,

the circuitry generates a current stimulus of selected wave form, amplitude, duration, polarity. In some embodiments, the circuitry causes an electrical repulsion of active agents in order to deliver the active agent to the biological subject.

In some embodiments, circuitry includes, among other things, one or more computing devices such as a processor (e.g., a microprocessor, a quantum processor, qubit processor, etc.), a central processing unit (CPU), a digital signal processor (DSP), an application-specific integrated circuit (ASIC), a field programmable gate array (FPGA), or the like, or any combinations thereof, and can include discrete digital or analog circuit elements or electronics, or combinations thereof. In some embodiments, a module includes one or more ASICs having a plurality of predefined logic components. In some embodiments, a module includes one or more FPGAs, each having a plurality of programmable logic components.

In some embodiments, circuitry includes one or more electric circuits, printed circuits, electrical conductors, electrodes, electrocautery electrodes, cavity resonators, conducting traces, ceramic patterned electrodes, electro-mechanical components, transducers, and the like.

In some embodiments, circuitry includes one or more components operably coupled (e.g., communicatively, electromagnetically, magnetically, ultrasonically, optically, inductively, electrically, capacitive coupled, wirelessly coupled, or the like) to each other. In some embodiments, circuitry includes one or more remotely located components. In some embodiments, remotely located components are operably coupled, for example, via wireless communication. In some embodiments, remotely located components are operably coupled, for example, via one or more communication modules, receivers, transmitters, transceivers, or the like.

In some embodiments, circuitry includes memory that, for example, stores instructions or information. Non-limiting examples of memory include volatile memory (e.g., Random Access Memory (RAM), Dynamic Random Access Memory (DRAM), or the like), non-volatile memory (e.g., Read-Only Memory (ROM), Electrically Erasable Programmable Read-Only Memory (EEPROM), Compact Disc Read-Only Memory (CD-ROM), or the like), persistent memory, or the like. Further non-limiting examples of memory include Erasable Programmable Read-Only Memory (EPROM), flash memory, or the like. In some embodiments, memory is coupled to, for example, one or more computing devices by one or more instructions, information, or power buses.

In some embodiments, circuitry includes one or more computer-readable media drives, interface sockets, Universal Serial Bus (USB) ports, memory card slots, or the like, and one or more input/output components such as, for example, a graphical user interface, a display, a keyboard, a keypad, a trackball, a joystick, a touch-screen, a mouse, a switch, a dial, or the like, and any other peripheral device. In some embodiments, a module includes one or more user input/output components that are operably coupled to at least one computing device configured to control (electrical, electromechanical, software-implemented, firmware-implemented, or other control, or combinations thereof) at least one parameter associated with, for example, determining one or more tissue thermal properties responsive to detected shifts in turn-ON voltage.

In some embodiments, circuitry includes a computer-readable media drive or memory slot that is configured to accept signal-bearing medium (e.g., computer-readable memory media, computer-readable recording media, or the like). In some embodiments, a program for causing a system to execute any of the disclosed methods can be stored on, for example, a computer-readable recording medium, a signal-bearing medium, or the like. Non-limiting examples of signal-bearing media include a recordable type medium such as a magnetic tape, floppy disk, a hard disk drive, a Compact Disc (CD), a Digital Video Disk (DVD), Blu-Ray Disc, a digital tape, a computer memory, or the like, as well as transmission type medium such as a digital or an analog communication medium (e.g., a fiber optic cable, a waveguide, a wired communications link, a wireless communication link (e.g., receiver, transmitter, transceiver, transmission logic, reception logic, etc.). Further, non-limiting examples of signal-bearing media include, but are not limited to, DVD-ROM, DVD-RAM, DVD+RW, DVD-RW, DVD-R, DVD+R, CD-ROM, Super Audio CD, CD-R, CD+R, CD+RW, CD-RW, Video Compact Discs, Super Video Discs, flash memory, magnetic tape, magneto-optic disk, MINIDISC, non-volatile memory card, EEPROM, optical disk, optical storage, RAM, ROM, system memory, web server, or the like.

In some embodiments, circuitry includes acoustic transducers, electroacoustic transducers, electrochemical transducers, electromagnetic transducers, electromechanical transducers, electrostatic transducers, photoelectric transducers, radioacoustic transducers, thermoelectric transducers, ultrasonic transducers, and the like.

In some embodiments, circuitry includes electrical circuitry operably coupled with a transducer (e.g., an actuator, a motor, a piezoelectric crystal, a Micro Electro Mechanical System (MEMS), etc.). In some embodiments, circuitry includes electrical circuitry having at least one discrete electrical circuit, electrical circuitry having at least one integrated circuit, or electrical circuitry having at least one application specific integrated circuit. In some embodiments, circuitry includes electrical circuitry forming a general purpose computing device configured by a computer program (e.g., a general purpose computer configured by a computer program which at least partially carries out processes and/or devices described herein, or a microprocessor configured by a computer program which at least partially carries out processes and/or devices described herein), electrical circuitry forming a memory device (e.g., forms of memory (e.g., random access, flash, read only, etc.)), electrical circuitry forming a communications device (e.g., a modem, communications switch, optical-electrical equipment, etc.), and/or any non-electrical analog thereto, such as optical or other analogs.

In some embodiments, circuitry includes one or more sensors configured to detect at least one physiological characteristic associated with a biological subject.

Having described the iontophoresis device and the circuitry, in some embodiments, an iontophoresis device comprises an electrode assembly including at least one electrode and a cosmetic composition; and circuitry is operably coupled to the electrode assembly and configured to concurrently generate at least a continuous direct current stimulus and a pulsed current stimulus to the same electrode, the continuous direct current stimulus and the pulsed current stimulus of a character and for a duration sufficient to deliver a cosmetic composition to a biological subject.

In some embodiments, during operation, the iontophoresis device includes circuitry configured to generate a continuous direct current stimulus having an average current density ranging from 0.01 $mA/cm^2$ to 0.5 $mA/cm^2$.

In some embodiments, the iontophoresis device includes circuitry configured to generate a continuous direct current stimulus having an average current density of 0.2 $mA/cm^2$.

In some embodiments, the iontophoresis device includes circuitry configured to generate a pulsed current stimulus having an average current density ranging from 0.01 $mA/cm^2$ to 10 $mA/cm^2$, a pulse width ranging from 50 microseconds to 1 milliseconds, and a pulse frequency ranging from 10 Hertz to 500 Hertz, and the duty cycle of pulses ranging from 1% to 90%.

In some embodiments, the iontophoresis device includes circuitry configured to generate a pulsed current stimulus having an average current density ranging from 0.01 $mA/cm^2$ to 10 $mA/cm^2$, a pulse width ranging from 50 microseconds to 1 milliseconds, at least one wave packet (or wave train) having from 2 to 20 pulses; a frequency of the wave packet ranging from 10 Hertz to 500 Hertz, and a duty cycle of pulses ranging from 1% to 90%.

In some embodiments, the iontophoresis device includes circuitry configured to generate a pulsed current stimulus having an average current density ranging from 0.01 $mA/cm^2$ to 10 $mA/cm^2$, a pulse width ranging from 50 microseconds to 1 milliseconds, at least one wave packet (or wave train) ranging from 2 to 20 pulses with alternating polarity, a frequency of the wave packet ranging from 10 Hertz to 500 Hertz, and a duty cycle of pulses ranging from 1% to 90%.

In some embodiments, the iontophoresis device includes circuitry configured to generate a pulsed current stimulus having an average current density of 0.2 $mA/cm^2$, alternating pulse duration of 500 microseconds, and a pulse frequency of 200 Hertz.

In some embodiments of the iontophoresis device, the electrode assembly includes at least one reservoir holding an aqueous active agent composition.

In some embodiments of the iontophoresis device, the electrode assembly includes at least one active electrode electrically coupled to a reservoir holding a cosmetic composition, particularly an active agent aqueous composition; the electrode assembly operable to transdermally deliver the aqueous active agent composition to a biological subject responsive to one or more inputs from the circuitry configured to concurrently generate the continuous direct current stimulus and the pulsed current stimulus.

In some embodiments of the iontophoresis device, the electrode assembly is electrically coupled to at least one power source.

In some embodiments of the iontophoresis device, the electrode assembly includes at least one active electrode assembly and at least one counter electrode assembly.

In some embodiments of the iontophoresis device, the electrode assembly includes at least one reservoir holding a cosmetic composition comprising a face care or body care composition, comprising, in particular, an active agent chosen from humectant or moisturizing active agents, anti-ageing active agents, for example depigmenting active agents, active agents that act on cutaneous microcirculation, or seboregulating active agents, or a composition for making up the face or body, or a hair composition, in particular, a composition for washing the hair, for hair care or conditioning, for temporary form retention or shaping of the hair, for the temporary, semi-permanent or permanent dyeing of the hair, or for relaxing or permanent waving, in particular, a composition for relaxing, dyeing or bleaching the roots and hair, or a composition for the scalp, in particular, an antidandruff composition, a composition for preventing hair loss or for promoting regrowth of the hair, an anti-seborrheic composition, an anti-inflammatory composition, an anti-irritation or soothing composition, a mark-preventing composition or a composition for stimulating or protecting the scalp.

FIGURES 2-4 illustrate embodiments of electrical stimuli of representative current density waveforms generated by the circuitry of the iontophoresis device to administer N-acetyl-3-trifluoromethyl-phenyl-(valine-glycine) or any other aqueous active agent composition.

Referring to FIGURE 2, a first current density waveform is illustrated for iontophoresis. FIGURE 2 illustrates one embodiment of a waveform that can be generated by the circuitry and iontophoresis device of FIGURE 1. FIGURE 1 shows a continuous direct current waveform stimulus. The current density waveform is controlled an average constant value for the duration or any part of the iontophoresis treatment. In some embodiments, a current density waveform controlled at a constant value is referred to as continuous direct current, and the terms "direct current," "DC current," "galvanic current," and "DC" are interchangeable.

In some embodiments, a positive current density means that the polarity at the first electrode 114 is positive.

The second electrode 116 has negative polarity when the first electrode has positive polarity. Following convention, this means that current flows from the first positive electrode 1114 to second negative electrode 116. Conversely, when the first electrode 114 has negative polarity and the second electrode 116 has positive polarity, current flows from the second positive electrode 116 to the first negative electrode 114, and this will be represented on a graph by a negative value of current density. Conventionally, electrons will be defined to flow in the opposite direction to current, i.e., from negative polarity to positive polarity. Current density is defined as ampere units per area units (of the cross section of the active electrode).

While FIGURE 2 depicts a certain continuous direct current density value and duration, it should be appreciated that the illustrated values are exemplary. In some embodiments of the continuous direct current waveform of FIGURE 2, the average current density is controlled at or below 0.5 $mA/cm^2$. In some embodiments of the continuous direct current waveform of FIGURE 2, the average current density is controlled at or below 0.2 $mA/cm^2$. In some embodiments of the continuous direct current waveform of FIGURE 2, the average current density is controlled between 0.01 $mA/cm^2$ to 0.5 $mA/cm^2$. In some embodiments of the continuous direct current waveform of FIGURE 2, the average current density is controlled at any one of the following values, 0.01, 0.05, 0.1, 0.15, 0.2, 0.25, 0.3, 0.35, 0.4, 0.45, 0.5 $mA/cm^2$ or in a range between any two values serving as endpoints. In some embodiments of the continuous direct current waveform of FIGURE 2, the amplitude is controlled at any of the above values and electrical current is applied for duration of at least 1 minute. In some embodiments of the continuous direct current waveform of FIGURE 2, the amplitude is controlled at any of the above values and electrical current is applied for duration of from 10 to 20 minutes. In some embodiments of the continuous direct current waveform of FIGURE 2, the amplitude is controlled at any of the above values and electrical current is applied for a duration (in minutes) of 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 30.5, 31, 31.5, 32, 32.5, 33, 33.5, 34, 34.5, 35 35.5, 36, 36.5, 37, 37.5, 38, 38.5, 39, 39.5, 40, 40.5, 41, 41.5, 42, 42.5, 43, 43.5, 44, 44.5, 45, 45.5, 46, 46.5, 47, 47.5, 48, 48.5, 49, 49.5, 50, 50.5, 51, 51.5, 52, 52.5, 53, 53.5, 54, 54.5, 55, 55.5, 56, 56.5, 57, 57.5, 58, 58.5, 59, 59.5, 60, or any range between any two values serving as endpoints. In some of the embodiments of the continuous direct current waveform of FIGURE 2, the first electrode 114 is positive and the second electrode 116 is negative for duration of the waveform.

Referring to FIGURE 3, a second current density waveform is illustrated for iontophoresis. FIGURE 3 illustrates one embodiment of a waveform that can be generated by the circuitry and the iontophoresis device of FIGURE 1. FIGURE 3 shows a pulse wave. The current density waveform is controlled in positive pulses (polarity is positive at electrode 114) for the duration or any part of the iontophoresis treatment. In some embodiments, the polarity can be reversed. The pulses of FIGURE 3 are unipolar, meaning that current travels in one direction. A pulse of FIGURE 3 has maximum amplitude. The pulse waveform will increase from a minimum amplitude, reach the maximum amplitude, and then decrease to the minimum amplitude, reside at the minimum amplitude, and the cycle will repeat. In some embodiments, a pulse is counted starting from the minimum amplitude, reaching the maximum amplitude, and then returning to the minimum amplitude. Thus, a pulse does not include the period of the minimum amplitude. In some embodiments, a pulse cycle does include the period at the minimum amplitude. In some embodiments, the durations of the maximum and minimum amplitudes are the same.

In some embodiments, the pulse wave is expressed to have a % duty cycle. In some embodiments, expressing a % duty cycle with respect to a pulse wave means that the electrical current is on for the % duty cycle. For example, 50 % duty cycle means electrical current is on for 50% and off for 50% of the pulse cycle, 30% duty cycle means electrical current is on for 30% and off for 70% of the pulse cycle. In some embodiments, the % duty cycle of unidirectional pulses is 5, 10, 15, 20, 25, 30, 35, 40, 45, 50, 55, 60, 65, 70, 75, 80, 85, 90, 95, or any range between any two values serving as endpoints. In some embodiments, the % duty cycle of a respective bipolar pulse is 0.01, 5, 10, 15, 20, 25, 30, 35, 40, 45, 50, 55, 60, 65, 70, 75, 80, 85, 90, 95, 100 or any range between any two values serving as endpoints. In some embodiments, the duty cycle of pulses ranges from 1% to 90%. In some embodiments, the pulse, meaning the "on" period can be expressed as a duration having units of time. In some embodiments, the pulse "off' period can be expressed as a duration. In some embodiments, the pulse wave will be expressed in hertz, meaning cycles per second.

In some embodiments, the pulses can be reversed by alternating the polarities of the first and second electrodes between negative and positive. In some embodiments, bipolar pulses, alternating pulses, bidirectional pulses, and reverse pulses mean the same. In some embodiments, negative current density pulses will be followed by positive current density pulses, without residing at a minimum. A pulse waveform including both negative and positive current density pulses will include a maximum value for negative pulses, a maximum value for positive pulses, and the values do not have to be the same. Further, in some embodiments, the duration of negative current density pulse does not have to be the same duration of a positive current density pulse. In some embodiments, the duration of pulses does not have to be the same duration, regardless whether the pulses are negative or positive.

In some embodiments, a pulse waveform can combine two or more pulse waveforms concurrently or alternatively. In some embodiments, a pulse waveform can include negative pulses, followed by positive pulses. Thus, having

a maximum and minimum amplitude for the negative pulses and a maximum and a minimum amplitude for the positive pulses.

While FIGURE 3 depicts certain current density values of the maximum and minimum pulse amplitudes and pulse duration, it should be appreciated that the illustrated values are exemplary only. In some embodiments of the current waveform of FIGURE 3, the average current density is controlled in pulses and each pulse maximum is controlled at most at 0.2 $mA/cm^2$ and the minimum amplitude is 0. In some embodiments of the current waveform of FIGURE 3, the average current density is controlled in pulses and each pulse maximum is controlled at or below 0.5 $mA/cm^2$ and the minimum amplitude is 0. In some embodiments of the current waveform of FIGURE 3, the average current density is controlled in pulses and each pulse maximum is controlled from 0.2 $mA/cm^2$ to 0.5 $mA/cm^2$ and the minimum amplitude is 0. In some embodiments of the current waveform of FIGURE 3, the average current density is controlled in pulses and each pulse maximum is controlled from 0.01 $mA/cm^2$ to 10 $mA/cm^2$ and the minimum amplitude is 0. In some embodiments of the current waveform of FIGURE 3, the average current density is controlled in pulses and each pulse maximum is controlled from 0.05 $mA/cm^2$ to 0.5 $mA/cm^2$ and the minimum amplitude is 0. In some embodiments of the current waveform of FIGURE 3, the average current density is controlled in pulses and each pulse maximum is controlled at or below 0.2 $mA/cm^2$ and the minimum amplitude is 0. In some embodiments of the current waveform of FIGURE 3, the average current density is controlled in pulses and each pulse maximum is controlled at 0.01, 0.05, 0.1, 0.15, 0.2, 0.25, 0.3, 0.35,0.4, 0.45, 0.5 $mA/cm^2$ or in the range between any two values serving as endpoints. In some embodiments, the current density is given as the root-mean-square (rms). In some embodiments, the current density is given as the average current density. In some embodiments, the current density can be given as the peak current density, which can be as high as 1 $mA/cm^2$ or 2mA/ $cm^2$ with a duty cycle of 50% and 25%, respectively.

In some embodiments, the pulse has a positive constant slope (other than vertical) to the maximum amplitude, followed by a duration at the constant maximum amplitude, followed by a negative constant slope (other than vertical) to 0, followed by a duration at 0. In some embodiments, the minimum can be other than 0. In some embodiments, the slope can be other than constant, such as exponential. In some embodiments of the current waveform of FIGURE 3, the pulses are not triangular. In some embodiments of FIGURE 3, the pulses are a square wave, wherein the maximum and the minimum amplitudes are of the same duration or not. In some embodiments of the current waveform of FIGURE 3, the pulses are not square wave. In some embodiments, the pulse wave is sinusoidal, non-sinusoidal, or any combination. In some embodiments, the pulse wave is periodic square wave, rectangular wave, saw tooth wave, spiked wave, trapezoidal wave, triangle wave, or combinations thereof.

In some embodiments of FIGURE 3, the duration of the maximum amplitude of the pulses is less than the duration of the minimum amplitude between pulses. In some embodiments of FIGURE 3, the pulse duration (or width) is defined as the time between the minimums with a maximum (either positive or negative) between the two minimums. In some embodiments, the pulse duration (or width) is given in units of time. In some embodiments, the pulse duration (or width) ranges from 50 microseconds to 1 milliseconds. In some embodiments, the pulse duration (or width) ranges from 200 microseconds to 300 microseconds. In some embodiments, the pulse duration (or width) ranges from 10 microseconds to 500 microseconds. In some embodiments, the pulse duration (or width) ranges from 50 microseconds to 5 milliseconds. In some embodiments, the pulse duration (or width) is less than 50 microseconds or greater than 5 milliseconds. In some embodiments, the pulse duration (or width) is 500 microseconds. In some embodiments of FIGURE 3, the duration of the maximum amplitude of the pulses is greater than the duration of the minimum amplitude between pulses.

In some embodiments of the current waveform of FIGURE 3, the minimum amplitude is 0 $mA/cm^2$.

In some embodiments of FIGURE 3, the minimum amplitude is greater than 0 $mA/cm^2$. In some embodiments of the current waveform of FIGURE 3, the maximum (and minimum) amplitude can increase from pulse to pulse. In some embodiments of the current waveform of FIGURE 3, the maximum (and minimum) amplitude can decrease from pulse to pulse. In some embodiments of the current waveform of FIGURE 3, the maximum (and minimum) amplitude can increase from pulse to pulse, and then decrease from pulse to pulse, and repeat.

In some embodiments of the current waveform of FIGURE 3, the average current density is controlled in pulses at any of the above values and the rate of pulses is from 100 hertz to 300 hertz. In some embodiments of the current waveform of FIGURE 3, the average current density is controlled in pulses at any of the above values and the rate of pulses is from 1 hertz to 200 hertz. In some embodiments, the pulse is less than 1 hertz. In some embodiments of the current waveform of FIGURE 3, the average current density is controlled in pulses at any of the above values and the rate of pulses is from 1 hertz to 500 hertz. In some embodiments of the current waveform of FIGURE 3, the average current density is controlled in pulses at any of the above values and the rate of pulses is 200 hertz. In some embodiments of the current waveform of FIGURE 3, the average current density is controlled in pulses at any of the above values and the rate of pulses is from 10 hertz to 500 hertz. In some embodiments of the current waveform of FIGURE 3, the average current density is controlled in pulses wherein the rate of pulses is from 1 hertz to 500 hertz, or any value in between in increments of 1 hertz.

In some of the embodiments of FIGURE 3, a pulse wave travels in wave packets (or wave trains). In some

embodiments, the wave packets are defined by the number of pulses, the duration or width of pulses in the packet, the average current density of pulses, the duty cycle of pulses in the wave packet, and the frequency of the wave packets. In some embodiments, the wave packet can have from 2 pulses or greater with or without alternating polarity. In some embodiments of FIGURE 3, the wave packet can have from 2 pulses to 20 pulses with or without alternating polarity. In some embodiments of FIGURE 3, the wave packets can be generated at a frequency from 10 Hertz or greater. In some embodiments of FIGURE 3, the wave packets can be generated at a frequency from 10 Hertz to 500 Hertz. In some embodiments, the duty cycle of the pulses in the wave packets ranges from 1% to 90%. In some embodiments, the pulses have an average current density of 0.01mA/$cm^2$ to 10 mA/$cm^2$.

In some embodiments of the current waveform of FIGURE 3, the iontophoresis treatment is applied for duration of from 1 minute to 5 minutes. In some embodiments of the current waveform of FIGURE 3, the iontophoresis treatment is applied for a duration (in minutes) of 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 30.5, 31, 31.5, 32, 32.5, 33, 33.5, 34, 34.5, 35 35.5, 36, 36.5, 37, 37.5, 38, 38.5, 39, 39.5, 40, 40.5, 41, 41.5, 42, 42.5, 43, 43.5, 44, 44.5, 45, 45.5, 46, 46.5, 47, 47.5, 48, 48.5, 49, 49.5, 50, 50.5, 51, 51.5, 52, 52.5, 53, 53.5, 54, 54.5, 55, 55.5, 56, 56.5, 57, 57.5, 58, 58.5, 59, 59.5, 60, or any range between any two values serving as endpoints. In some embodiments of the current waveform of FIGURE 3, the first electrode 114 has positive polarity and the second electrode 116 has negative polarity when the pulses extend above 0. However, the first electrode 114 is negative and the second electrode 116 is positive when the pulses extend below 0. Thus, indicating a reversal in the direction of current.

Referring to FIGURE 4, a third current density waveform is illustrated for iontophoresis. FIGURE 4 illustrates one embodiment of a waveform that can be generated by the iontophoresis device of FIGURE 1. FIGURE 4 shows a continuous direct current concurrent with a pulse wave. The current density waveform is controlled in positive unipolar pulses and between pulses; the current density is controlled as continuous direct current. Stated another way, the current waveform of FIGURE 4 can be described as the combination between a direct current and a bidirectional pulse taken as an offset direct current with a duty cycle smaller than 100%. The two waveforms are applied concurrently for the duration or any part of the iontophoresis treatment. Specifically, the pulse wave has an on and off period. The superposition of the pulse wave on top of continuous direct current causes the current profile to show the pulse beginning at the constant value of the direct current. The pulse reaches a maximum for the predetermined duration and then the profile goes to 0. After the off period from the 0 value, the continuous direct current is applied until then next pulse. Thus, the current density of the waveform can be described as the addition of continuous direct current of first amplitude with a pulse of second amplitude, wherein the pulse has an off period before applying the direct current again. A pulse is counted starting from the direct current amplitude, reaching the maximum pulse amplitude, and then returning to the minimum amplitude or 0. Thus, a pulse does not include the period of the minimum amplitude at 0. A pulse cycle does include the period at the minimum amplitude. In some embodiments, the durations of the maximum and minimum amplitudes are the same.

In some embodiments, the pulse is expressed to have a % duty cycle. In some embodiments, expressing a % duty cycle with respect to a pulse wave means that the electrical current is on for the % duty cycle. For example, 50 % duty cycle of pulses means electrical current is on for 50% and off for 50% of the pulse cycle, 30% duty cycle of pulses means electrical current is on for 30% and off for 70% of the pulse cycle. In some embodiments, the % duty cycle of unidirectional pulses is 5, 10, 15, 20, 25, 30, 35, 40, 45, 50, 55, 60, 65, 70, 75, 80, 85, 90, 95, or any range between any two values serving as endpoints. In some embodiments, the % duty cycle of a respective bipolar pulse is 0.01, 5, 10, 15, 20, 25, 30, 35, 40, 45, 50, 55, 60, 65, 70, 75, 80, 85, 90, 95, 100 or any range between any two values serving as endpoints. In some embodiments, the duty cycle of pulses ranges from 1% to 90%. In some embodiments, the pulse, meaning the "on" period can be expressed as a duration. In some embodiments, the pulse "off" period can be expressed as a duration. In some embodiments, the pulse wave is expressed in hertz, meaning cycles per second. In some embodiments, the pulses can be reversed by alternating the polarities of the first and second electrodes between negative and positive. In some embodiments, bipolar pulses, alternating pulses, bidirectional pulses, and reverse pulses mean the same. In some embodiments, negative current density pulses will be followed by positive current density pulses, without residing at a minimum. A pulse waveform including both negative and positive current density pulses will include a maximum value for negative pulses, a maximum value for positive pulses, and the values do not have to be the same. Further, in some embodiments, the duration of negative current density pulse does not have to be the same duration of a positive current density pulse. In some embodiments, the duration of pulses does not have to be the same duration, regardless whether the pulses are negative or positive.

In some embodiments, a pulse waveform can combine two or more pulse waveforms concurrently or alternatively. In some embodiments, a pulse waveform can include negative pulses, followed by positive pulses. Thus, having maximum and minimum amplitude for the negative ,pulses and maximum and minimum amplitude for the positive pulses.

While FIGURE 4 depicts certain current density values of the maximum and minimum pulse amplitudes and pulse duration, it should be appreciated that the illustrated values are exemplary only. In some embodiments of the

current waveform of FIGURE 4, concurrent with a direct current, the average current density is controlled in pulses and each pulse maximum is controlled at most at 0.2 mA/cm$^2$ and the minimum amplitude is 0. This means that the addition of the pulse to the direct current totals 0.4 mA/cm$^2$. In some embodiments of the current waveform of FIGURE 4, concurrent with a direct current, the average current density is controlled in pulses and each pulse maximum is at or below 0.5 mA/cm$^2$ and the minimum amplitude is 0, and direct current average current density is controlled at or below an average of 0.5 mA/cm$^2$. In some embodiments of the current waveform of FIGURE 4, concurrent with a direct current, the average current density is controlled in pulses and each pulse maximum is controlled from 0.2 mA/cm$^2$ to 0.5 mA/cm$^2$ and the minimum amplitude is 0, and the direct current average current density is controlled from 0.2 mA/cm$^2$ to 0.5 mA/cm$^2$. In some embodiments of the current waveform of FIGURE 4, concurrent with a direct current, the average current density is controlled in pulses and each pulse maximum is controlled at or below 0.2 mA/cm$^2$ and the minimum amplitude is 0, and the direct current average current density is controlled at or below 0.2 mA/cm$^2$. In some embodiments of the current waveform of FIGURE 4, concurrent with direct current, the average current density is controlled in pulses and each pulse maximum is controlled from 0.01 mA/cm$^2$ to 10 mA/cm$^2$, and the direct current average current density is controlled from 0.01 mA/cm$^2$ to 0.5 mA/cm$^2$, In some embodiments of the current waveform of FIGURE 4, concurrent with direct current, the average current density is controlled in pulses and the direct current and each pulse maximum is controlled on average at 0.01, 0.05, 0.1, 0.15, 0.2, 0.25, 0.3, 0.35,0.4, 0.45, 0.5 mA/cm$^2$ or in the range between any two values serving as endpoints. In some embodiments, the current density is given as the root-mean-square (rms). In some embodiments, the current density is given as the average current density. In some embodiments, the current density can be given as the peak current density, which can be as high as 1 mA/cm$^2$ or 2mA/ cm$^2$ with a duty cycle of 50% and 25%, respectively.

In some embodiments of FIGURE 4, the pulses are triangular with a defined maximum and minimum, wherein the maximum and the minimum amplitudes are of the same duration. Specifically, the pulse has a positive constant slope (other than vertical) to the maximum amplitude, followed by a period at the constant maximum amplitude, followed by a negative constant slope (other than vertical) to 0, followed by a period at 0. In some embodiments, the minimum can be other than 0. In some embodiments, the slope can be other than constant, such as exponential. In some embodiments, the pulse wave is sinusoidal, non-sinusoidal, or any combination. In some embodiments, the pulse wave is periodic square wave, rectangular wave, saw tooth wave, spiked wave, trapezoidal wave, triangle wave, or combinations thereof.

In some embodiments of FIGURE 4, the duration of the maximum amplitude of the pulses is less than the duration of the minimum amplitude between pulses. In some embodiments of FIGURE 4, the pulse duration (or width) is defined as the time between the minimums with a maximum (either positive or negative) between the two minimums. In some embodiments, the pulse duration (or width) is given in units of time. In some embodiments, the pulse duration (or width) ranges from 50 microseconds to 1 milliseconds. In some embodiments, the pulse duration (or width) ranges from 200 microseconds to 300 microseconds. In some embodiments, the pulse duration (or width) ranges from 10 microseconds to 500 microseconds. In some embodiments, the pulse duration (or width) ranges from 50 microseconds to 5 milliseconds. In some embodiments, the pulse duration (or width) is less than 50 microseconds or greater than 5 milliseconds. In some embodiments, the pulse duration (or width) is 500 microseconds. In some embodiments of FIGURE 4, the duration of the maximum amplitude of the pulses is greater than the duration of the minimum amplitude between pulses.

In some embodiments of the current waveform of FIGURE 4, the minimum amplitude is 0 mA/cm$^2$. In some embodiments of FIGURE 4, the minimum amplitude is greater than 0 mA/cm$^2$. In some embodiments of the current waveform of FIGURE 4, the maximum (and minimum) amplitude can increase from pulse to pulse. In some embodiments of the current waveform of FIGURE 4, the maximum (and minimum) amplitude can decrease from pulse to pulse. In some embodiments of the current waveform of FIGURE 4, the maximum (and minimum) amplitude can increase from pulse to pulse, and then decrease from pulse to pulse, and repeat.

In some embodiments of the current waveform of FIGURE 4, the average current density is controlled as direct current concurrently with pulses at any of the above values and the rate of pulses is from 100 hertz to 300 hertz. In some embodiments of the current waveform of FIGURE 4, the current density is controlled as direct current concurrently with pulses at any of the above values and the rate of pulses is from 1 hertz to 500 hertz. In some embodiments of the current waveform of FIGURE 4, the average current density is controlled as direct current concurrently with pulses wherein the rate of pulses is from 1 hertz to 500 hertz, or any value in between in increments of 1 hertz. In some embodiments of the current waveform of FIGURE 4, concurrent with direct current, the average current density is controlled in pulses at any of the above values and the rate of pulses is from 10 hertz to 500 hertz. In some embodiments of the current waveform of FIGURE 4, each pulse has a duration of between 0.001 seconds to 1 second or any value in between. In some embodiments of the current waveform of FIGURE 4, concurrent with direct current, the average current density is controlled in pulses and the rate of pulses is 200 hertz and each pulse has a duration of 500 microseconds.

In some of the embodiments of FIGURE 4, the pulses are applied as a wave packet (or wave train). In some embodiments, the wave packets are defined by the number of pulses, the duration or width of pulses in the packet, the

average current density of pulses, the duty cycle of pulses in the wave packet, and the frequency of the wave packets. In some embodiments, the wave packet can have from 2 pulses or greater with or without alternating polarity. In some embodiments of FIGURE 4, the wave packet can have from 2 pulses to 20 pulses with or without alternating polarity. In some embodiments of FIGURE 4, the wave packets can be generated at a frequency from 10 Hertz or greater. In some embodiments of FIGURE 4, the wave packets can be generated at a frequency from 10 Hertz to 500 Hertz. In some embodiments, the duty cycle of the pulses in the wave packets ranges from 1% to 90%. In some embodiments, the pulses of wave packets have an average current density of 0.01mA/$cm^2$ to 10 mA/$cm^2$.

In some embodiments of the current waveform of FIGURE 4, the iontophoresis treatment is applied for a duration (in minutes) of 1, 1.5, 2, 2.5, 3, 3.5, 4, 4.5, 5 or any range between any two values serving as endpoints. In some embodiments of the waveform of FIGURE 4, the electrical current is applied as continuous direct current and in pulses for a duration (in minutes) of 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 30.5, 31, 31.5, 32, 32.5, 33, 33.5, 34, 34.5, 35 35.5, 36, 36.5, 37, 37.5, 38, 38.5, 39, 39.5, 40, 40.5, 41, 41.5, 42, 42.5, 43, 43.5, 44, 44.5, 45, 45.5, 46, 46.5, 47, 47.5, 48, 48.5, 49, 49.5, 50, 50.5, 51, 51.5, 52, 52.5, 53, 53.5, 54, 54.5, 55, 55.5, 56, 56.5, 57, 57.5, 58, 58.5, 59, 59.5, 60, or any range between any two values serving as endpoints. In some embodiments of the current waveform of FIGURE 4, the pulses are unipolar pulses.

In some embodiments of the current waveform of FIGURE 4, the first electrode 114 has positive polarity and the second electrode 116 has negative polarity when the pulses extend above 0. However, the first electrode 114 is negative and the second electrode 116 is positive when the pulses extend below 0. Thus, indicating a reversal in the direction of current.

In some embodiments, the current waveforms of FIGURES 2, 3 and 4 can be combined to provide concurrent waveforms for the administration of aqueous active agent compositions, such as N-acylaminoamide inhibitor of elastase compositions, and in particular N-acetyl-3-trifluoromethyl-phenyl-(valine-glycine) compositions, into the skin via the use of iontophoresis. In some embodiments, the current waveforms of FIGURES 2, 3 and 4 can be combined to provide concurrent waveforms for the administration of one or more of a face care or body care composition, comprising, in particular, an active agent chosen from humectant or moisturizing active agents, anti-ageing active agents, for example depigmenting active agents, active agents that act on cutaneous microcirculation, or seboregulating active agents, a composition for making up the face or body, a hair composition, in particular, a composition for washing the hair, for hair care or conditioning, for temporary form retention or shaping of the hair, for the temporary, semi-permanent or permanent dyeing of the hair, or for relaxing or permanent waving, in particular, a composition for relaxing, dyeing or bleaching the roots and hair, and a composition for the scalp, in particular, an antidandruff composition, a composition for preventing hair loss or for promoting regrowth of the hair, an anti- seborrheic composition, an anti-inflammatory composition, an anti-irritation or soothing composition, a mark-preventing composition or a composition for stimulating or protecting the scalp.

In some embodiments, the current waveforms of FIGURES 2, 3 and 4 can be combined to provide combinations of waveforms that are generated by the circuitry of the iontophoresis device of FIGURE 1. In some embodiments, the current waveform of any FIGURE 2-4 is applied for a first duration, followed by a different current waveform of any FIGURE 2-4 for a second duration or vice versa. In some embodiments two or more different current waveforms can be cycled for the entire electrical current treatment duration. The particulars of the waveform as described above for FIGURES 2-4 are similarly applicable to combined treatments applying the two or more different waveforms. That is, any one or more of the embodiments of the direct current waveform can be combined with any one or more of the pulse waveforms in sequence or simultaneously.

In the case of the waveforms of FIGURES 2, 3, and 4, the peak voltage at maximum peak is 99 volts. In some embodiments, the maximum duration of conducting electrical current is 120 minutes during the iontophoresis treatment.

Every embodiment of the current density waveforms described in connection with FIGURES 2, 3 and 4 and the combination of waveforms can be used for iontophoresis of every embodiment of the compositions.

## COMPOSITION

A iontophoresis composition for use with the iontophoresis methods described above is disclosed.

In some embodiments, the iontophoresis composition includes one or more of N-acylaminoamide inhibitor of elastase, and in particular one or more of N-acetyl-3-trifluoromethyl-phenyl-(valine-glycine), N-acetyl-3-trifluoromethyl-phenyl-(valine-glycine) derivatives, ions of N-acetyl-3-trifluoromethyl-phenyl-(valine-glycine), and ions of N-acetyl-3-trifluoromethyl-phenyl-(valine-glycine) derivatives present in amounts ranging from 0.01 % to 30 % by weight; and water present in an amount of at least 20 % by weight; the iontophoresis composition having an aqueous phase that is at least 30% by weight relative to the total weight of the iontophoresis composition.

In some embodiments, the iontophoresis composition includes one or more of N-acylaminoamide inhibitor of

elastase, and in particular one or more of N-acetyl-3-trifluoromethyl-phenyl-(valine-glycine), N-acetyl-3-trifluoromethyl-phenyl-(valine-glycine) derivatives, ions of N-acetyl-3-trifluoromethyl-phenyl-(valine-glycine), and ions of N-acetyl-3-trifluoromethyl-phenyl-(valine-glycine) derivatives present in amounts ranging from 0.1 % to 30 % by weight; one or more silicon materials present in amounts ranging from 0.1 % to 30 % by weight; and water present in an amount of at least 20 % by weight; the iontophoresis composition having an aqueous phase that is at least 30% by weight relative to the total weight of the iontophoresis composition.

In some embodiments, the iontophoresis composition further comprises one or more ionic polymers present in amounts ranging from 0.01 % to 10% by weight; wherein the one or more of N-acylaminoamide inhibitor of elastase, and in particular one or more of N-acetyl-3-trifluoromethyl-phenyl-(valine-glycine), N-acetyl-3-trifluoromethyl-phenyl-(valine-glycine) derivatives, ions of N-acetyl-3-trifluoromethyl-phenyl-(valine-glycine), and ions of N-acetyl-3-trifluoromethyl-phenyl-(valine-glycine) derivatives are present in amounts ranging from 0.1 % to 30 % by weight.

In some embodiments, the iontophoresis composition further comprises one or more non-ionic polymers present in amounts ranging from 0.01 % to 20% by weight; wherein the one or more of N-acylaminoamide inhibitor of elastase, and in particular one or more of N-acetyl-3-trifluoromethyl-phenyl-(valine-glycine), N-acetyl-3-trifluoromethylphenyl-(valine-glycine) derivatives, ions of N-acetyl-3-trifluoromethyl-phenyl-(valine-glycine), and ions of N-acetyl-3-trifluoromethyl-phenyl-(valine-glycine) derivatives are present in amounts ranging from 0.01 % to 30 % by weight.

In some embodiments, the iontophoresis composition further comprises a pH ranging from 2 to 7.5.

In some embodiments of the iontophoresis composition, the one or more silicon materials include one or more silicon surface-active agents. In some embodiments of an iontophoresis composition, the silicon-containing surface active agents are selected from polydimethylsiloxane, poly[oxy(dimethylsilylane)], polyvinyl siloxane, cyclohexasiloxane, derivatives thereof, or any combination thereof.

In some embodiments of the iontophoresis composition, the ionic polymers and nonionic polymers are selected from acrylonitrile/methyl methacrylate/vinylidene chloride copolymer, sodium acrylate/sodium acryloyldimethyl taurate copolymer (and) isohexadecane (and) polysorbate 80, biosaccharide gum-1, sodium styrene/maleic anhydride copolymer, xanthan gum, ammonium polyacryloyldimethyl taurate, derivatives thereof, their ions, and any combination thereof.

In some embodiments of the iontophoresis composition, the composition further comprises a vitamin, a fat, a solvent, a humectant, a viscosity reducer, a preservative, a chelating agent, a viscosity controller, a skin conditioner, an emollient, an emulsifier, a cleansing agent, an emulsion stabilizer, a viscosity increaser, an antioxidant, a binder, a skin bleaching agent, a pH adjuster, a buffering agent, a denaturant, a bulking agent, an opacifying agent.

In some embodiments of the iontophoresis composition, the composition includes ionic polymers and nonionic polymers selected from biosaccharide gum-1 (and) sodium levulinate (and) glyceryl caprylate (and) sodium anisate, acrylates/c10-30 alkyl acrylate crosspolymer, sodium acrylate/sodium acryloyldimethyl taurate copolymer (and) isohexadecane (and) polysorbate 80, carbomer, sodium styrene/maleic anhydride copolymer, nylon-12, xanthan gum, derivatives thereof, their ions, or any combination thereof.

In some embodiments, the iontophoresis composition has a pH from 2 to 7.4.

In some embodiments, the iontophoresis composition has a pH from 2 to 7.

In some embodiments, the iontophoresis composition has a pH from 5.7 to 6.3.

In some embodiments, the iontophoresis composition has a pH from 2 to 6.3.

In some embodiments, the iontophoresis composition includes one or more vitamins selected from vitamin B5, vitamin A, vitamin B3, and vitamin E.

In some embodiments, the iontophoresis composition includes one or more fats selected from nut oils, seed oils, and plant oils.

In some embodiments, the iontophoresis composition includes one or more solvents selected from water, deionized water, Eau de la Roche-Posay™, ethanol.

In some embodiments, the iontophoresis composition includes one or more humectants selected from glycerin, caprylyl glycol, and sodium hyaluronate.

In some embodiments, the iontophoresis composition includes one or more viscosity reducers selected from glycerine.

In some embodiments, the iontophoresis composition includes one or more preservatives selected from phenoxyethanol, salicylic acid, and sodium methylparaben.

In some embodiments, the iontophoresis composition includes one or more chelating agents selected from disodium EDTA.

In some embodiments, the iontophoresis composition includes one or more viscosity controllers selected for instance from disodium EDTA, ammonium polyacryldimethyltauramide, and nylon-12.

In some embodiments, the iontophoresis composition includes one or more skin conditioners selected from C12-15 alkyl benzoate, caprylyl glycol, glyceryl stearate and polyethylene glycol 100 Stearate, tocopheryl acetate, sodium hyaluronate, ethylhexyl palmitate, dimethicone and dimethiconol, dimethicone, dimethicone and dimethicone/vinyl dimethicone crosspolymer, biosaccharide gum-1, oxothiazolidinecarboxylic acid, ascorbic acid, sodium styrene/maleic anhydride copolymer, salicylic acid, cyclohexasiloxane, hydrogenated polyisobutene, biosaccharide gum-1 and sodium levulinate and glyceryl caprylate and sodium anisate, lemon extract, alcohol and Gentiana lutea root extract, and dimethicone and polyethylene glycol/polypropylene glycol-18/18 dimethicone.

In some embodiments, the iontophoresis composition includes one or more emollients selected from C12-15 alkyl benzoate, caprylyl glycol, glyceryl stearate and polyethylene glycol 100 Stearate, ethylhexyl palmitate, dimethicone and dimethiconol, dimethicone, dimethicone and dimethicone/vinyl dimethicone crosspolymer, cyclohexasiloxane, hydrogenated polyisobutene, biosaccharide gum-1 and sodium levulinate and glyceryl caprylate and sodium anisate, and dimethicone and polyethylene glycol/polypropylene glycol-18/18 dimethicone.

In some embodiments, the iontophoresis composition includes one or more emulsifiers selected from glyceryl stearate and polyethylene glycol 100 Stearate, cetyl alcohol, xanthan gum, triethanolamine, biosaccharide gum-1 and sodium levulinate and glyceryl caprylate and sodium anisate, and dimethicone and polyethylene glycol/polypropylene glycol-18/18 dimethicone.

In some embodiments, the iontophoresis composition includes one or more cleansing agents selected from glyceryl stearate and polyethylene glycol 100 Stearate.

In some embodiments, the iontophoresis composition includes one or more stabilizers selected from cetyl alcohol, xanthan gum, ammonium polyacryldimethyltauramide, sodium styrene/maleic anhydride copolymer, carbomer, and acrylates/C10-30 alkylacrylate crosspolymer.

In some embodiments, the iontophoresis composition includes one or more viscosity increasers selected from cetyl alcohol, xanthan gum, dimethicone and dimethicone/vinyl dimethicone crosspolymer, carbomer, and acrylates/C10-30 alkylacrylate crosspolymer.

In some embodiments, the iontophoresis composition includes one or more pH adjusters selected from triethanolamine, potassium hydroxide, and sodium hydroxide.

In some embodiments, the iontophoresis composition includes one or more buffering agents selected from potassium hydroxide and hydroxyethylpiperazine ethane sulfonic acid, and sodium hydroxide.

In some embodiments, the iontophoresis composition includes one or more denaturants selected from sodium hydroxide.

In some embodiments, the iontophoresis composition includes one or more bulking agents selected from nylon-12.

In some embodiments, the iontophoresis composition includes one or more opacifying agents selected from nylon-12.

The following are representative components of a 1% by weight N-acetyl-3-trifluoromethyl-phenyl-(valine-glycine) composition suitable for use with all the embodiments of current waveforms in iontophoresis treatments. The amounts are given in weight percentages. The component names follow the International Nomenclature of Cosmetic Ingredients (INCI US).

Water (a solvent): 73.19% (range of 30% to 80%, and any value in between);
Glycerine (a humectant and viscosity reducer):7% (range of 0% to 10%, and any value in between);
Phenoxyethanol (a preservative): 0.5% (range of 0% to 1%, and any value in between);
Caprylyl glycol (a skin conditioner, emollient, and humectant): 0.7% (range of 0% to 1%, and any value in between);
PEG-30 dipolyhydroxyestearate (a skin conditioner, and agent): 0.5% (range of 0% to 5%, and any value in between);
Dimethicone crosspolymer (a skin conditioner, emollient and viscosity increaser): 2% (range of 0% to 5%, and any value in between);
Acetyl trifluoromethylphenyl valyclycine (an active): 1% (range of 0.1% to 25%, and any value in between);
Sodium hydroxide (a buffering agent and pH adjuster): 0.11% (range of 0% to 10%, and any value in between);
Sodium acrylate/sodium acryloyldimethiyl taurate copolymer (and) isohexadecane (and) polysorbate 80 (a thickener, stabilizer and texturizing polymer -5% (range of 0% to 10%, and any value in between);
Cyclohexasiloxane (silicon-containing surface active agent): 6% (range of 0% to 10%, and any value in between)
pH 5.7 to 6.3 and any value in between.

## EXAMPLES

The N-acetyl-3-trifluoromethyl-phenyl-(valine-glycine) has been used in a composition having simplex formulation of 1% (w/v) in water and/or polar solvent.

In the following, the "active agent" is N-acetyl-3-trifluoromethyl-phenyl-(valine-glycine).

In the following, FLUIDANOV™ is FLUIDANOV™.

Experimental Procedures

1. Preparation of skin for permeation study

Untreated porcine ears were procured from a local slaughter house. Dorsal side of the porcine ears was used for the study.. For study #4, dermatomed porcine skin was prepared by dermatoming full thickness skin using Dermatome 75 mm (Nouvag AG TCM 3000, Goldach, Switzerland).

Dermatome is a surgical instrument used to produce thin slices of skin. Dermatoming is refering to the procedure to produce thin layers of the skin by using such device.

2. Preparation of Franz cells for the study

In vitro permeation study was performed using Franz diffusion cells (PermeGear V6 station vertical cell). These models contain 6 diffusion cells in each console with 5 ml receptor volume and 9 mm orifice. The diffusion area was 0.64 $cm^2$.

Prior to each experiment, Franz cells were washed as follows:

3 times with Ethanol
3 times with deionized water
3 times with receptor solution (as specified for different studies in Table 2)

After washing the cells, receptor solution was left overnight and replaced with fresh sodium chloride solution (0.9 % w/v), 30 minutes prior to mounting the skin. The receptor chamber temperature was maintained at 37°C by the built-in water circulation jacket so as to attain the skin temperature of 32°C.

3. Mounting of skin over Franz diffusion cells

After thawing, skin was cut into pieces of appropriate sizes and their thickness was measured using digital micrometer (Electronic Equipt Co. Inc, Cedarhurst, NY, USA). The thickness values of the skin pieces used in different studies were measured.

Skin pieces were then mounted between the donor and receptor compartment (containing 0.9 % (w/v) sodium chloride solution) of Franz diffusion cells with the stratum corneum facing towards the donor side. The actual skin temperature was measured using IR thermometer and was found to be 31-32 °C in all the permeation studies.

4. Skin integrity testing

The barrier integrity of skin was evaluated non-invasively by skin electrical resistance assessment. Resistance was assessed by passing a constant current (voltage of 100 mV AC electrical field at 10 Hz, duty cycle 50% without offset) through skin membrane using Agilent 34410A 61/2 Digit Digital Multimeter and Agilent 33220A 20 MHz Function/Arbitrary waveform generator (Agilent Technologies, CA, USA).

5. In vitro permeation study

5.1. Application of test formulations

The formulation (160 mg formulation containing 1.6 mg active/0.64 sq.cm) was applied in the donor chamber using a positive displacement pipette (Rainin PosD). Simplex formulation was applied in the donor chamber. The donor compartment was left open to mimic in vivo conditions. Six replicates (with the exception of preliminary study 4) were performed for each formulation for each test group (iontophoresis and corresponding passive control - "no current" group) as specified in Table 1 as follows. In the Table 1, the "active agent" is N-acetyl-3-trifluoromethyl-phenyl-(valine-glycine).

Table 1

| **Study** | **Formulation** | **No. of cells** | **Type of iontophoresis and specifications** | **Formulatio n Applied (dose/0.6 4 sq.cm)** | **Receptor compositi on** | **Skin** |
|---|---|---|---|---|---|---|
| **#1** | FLUIDANOV™ + 1% active agent | 12 (6 Passive, 6 iontophoresis) | Cathodal, 0.2 mA/sq.cm for 20 min | 160 mg | PBS, pH 7.4 | Full thickness porcine ear skin |
| **#2** | Simplex: 1 % (w/v) active agent in water: ethanol (1:1) | 12 (6 Passive, 6, iontophoresis) | Cathodal, 0.2 mA/sq.cm for 20 min | 160 μL (1.6 mg) | PBS, pH 7.4 | Full thickness porcine ear skin |
| **#3** | Modified simplex: 1 % (w/v) active agent in PBS, pH 7.4: ethanol (1:1) | 12 (6 Passive, 6 iontophoresis) | Cathodal, 0.2 mA/sq.cm for 20 min | 160 μL | PBS, pH 7.4 | Full thickness porcine ear skin |
| #4 | 1 % (w/v) active agent in HEPES buffer with NaCl, pH 7.15: ethanol - (1:1) | 12 (4 Passive, 4 anodal, and 4 cathodal iontophoresis) | Cathodal and anodal, 0.2 mA/sq.cm for 2 h | 160 μL | HEPES buffer (pH 7.15) containing NaCl for passive and cathodal and without sodium chloride for anodal iontophores is | Dermatomed porcine ear skin |
| #5 | Modified simplex: 1 % (w/v) active agent in PBS, pH 7.4: ethanol (1:1) | 6 | Anodal, 0.2 mA/sq.cm for 20 min | 160 μL | PBS, pH 7.4 | Full thickness porcine ear skin |
| #6 | FLUIDANOV™ + 1% active agent | 6 | Anodal, 0.2 mA/sq.cm for 20 min | 160 mg | PBS, pH 7.4 | Full thickness porcine ear skin |

5.2. Application of iontophoresis

Current (0.2 mA/sq.) was applied using silver/silver chloride electrode system and a constant current source (Keithley®; Keithley Instruments, Cleveland, OH, USA). For cathodal iontophoresis, cathode (silver chloride) was placed in the donor and anode (silver wire) was placed in the receiver compartment and it was vice versa for anodal iontophoresis. Current application was monitored with a chronometer. In all the studies, current was applied for 20 min except study #4, where it was applied for 2 h.

5.3. Receptor sampling

Receptor samples were taken after stopping the current for the defined duration, which was 20 min in all the studies except study #4, in which sampling was performed at 5, 10, 20, 60, 120, and 240 min. Equal volume of fresh receptor solution was replaced after each sample was taken in case of multiple sampling time points. The receptor samples were filtered and quantified with methanol calibration using LCMS.

6. Unabsorbed formulations

At the end of the permeation study, skin was wiped with soft tissue to remove residual formulation followed by addition of lauryl ether sulfate 5% (LES) over the diffusion area which was then wiped by one cotton bud (for 30s in circular motion). Then, LES was added to another cotton bud which was used to wipe the skin surface, followed by three rinses, each with 1 ml of water. Finally, skin was dried by two cotton buds.

7. Skin distribution study

Skin stripping was done using D-Squame Stripping Discs and D-Squame pressure instrument. The first 5 tape strips were extracted and analyzed separately and then tape strips were pooled in multiples of five (tapes 6-10, 11-15, and 16-20) for extraction and assay. 5. Samples were quantified with methanol calibration after filtration using 0.45 μm Millipore membrane nylon filters. After tape stripping, the remaining skin was extracted as well. Skin was minced and 5mL methanol was added followed by mixing using Vortex followed by ultrasound for 10 min. Unabsorbed formulation as well as distribution of active in skin was not quantified in study #2 and #4. Only receptor samples were analyzed in these studies and the reason for the same is discussed in the following.

8. Quantitative analysis

All samples were analyzed by a validated LCMS method which was performed on an Agilent 6410 Triple Quad System to determine how much of the cosmetic agent passed into and through the skin.

8.1. Standard and quality control samples preparation

Master stock solutions were prepared individually by dissolving the accurately weighed reference substance (active agent) in methanol. Working calibrators were prepared by serial dilution with methanol. The working quality control (QC) samples were prepared independently QC working solutions were prepared in the same manner as the working calibrators.

8.2. Analytical procedures

HPLC was performed using Agilent 1200 system (Agilent, USA), comprising of system controller, quaternary gradient pump, auto sampler, column oven, and online degasser. Active agent was separated on a Zorbax Eclipse Plus C18 column (50 × 2.1 mm, 1.8 μm, Agilent, USA) with a Zorbax Eclipse Plus C18 security guard column (5 × 2.1 mm, 1.8 μm). Column oven temperature was set at 45 °C. A flow rate of 0.4 mL/min was used. The mobile phase consisted of A: water with 0.1% (v/v) of formic acid, and B: acetonitrile with 0.1% (v/v) of formic acid.

The HPLC system was coupled to an Agilent 6410 triple quadrupole mass spectrometer equipped with an electrospray ionization source (Agilent, USA). Active agent was monitored in positive electrospray mode with MRM transitions of 361.2 → 216.1 and 361.2 → 286.1, which served as the quantifier and the qualifier, respectively. Mass spectrometer parameters were as follows: Fragmentor=85, Collision energy=28 (361.2 → 216.1) and 4 (361.2 → 286.1), Dwell=200, Cell accelerator voltage=7 V, Gas temperature= 300 °C, Gas flow= 11 L/min, Nebulizer= 15 psi, Capillary voltage: 4000 V.

9. Data analysis

Results of the studies were plotted as average amount of active permeated in receptor, quantity of the active in tape strips, and underlying skin for each test group (passive or iontophoresis treatment group). All values have been presented as mean ± SE. Unpaired Student's t-test was performed using Microsoft Excel to investigate significant difference between two groups. Statistically significant difference was shown by p values < 0.05.

**Results**

**1. Study #1: Comparison of passive and cathodal iontophoretic delivery of active agent**

This study was performed to investigate the effect of cathodal iontophoresis (0.2 mA/sq.cm) applied for 20 min, on the delivery of the active agent from formulation FLUIDANOV™ + 1 % active agent, into and through full thickness porcine skin.

Fig. 5. Shows the amount of active in A. Receptor B. Stratum corneum (tape strips) and C. Skin (viable epidermis

and dermis).

However, no statistically significant difference ($p>0.05$) between the amount/sq.cm of the active in the different compartments (receptor, stratum corneum (tapes), and skin) was observed when the two test groups (passive and iontophoresis) were compared. The results clearly showed that 20 min cathodal iontophoresis was not able to improve the intradermal delivery of active agent from the given formulation (FLUIDANOV™ + 1% active agent). One of the reasons for ineffectiveness of iontophoresis in enhancing the delivery of the active under the specified conditions, was speculated to be due to the complex composition of the test formulation. Hence, our next aim was to investigate the effect of 20 min cathodal iontophoresis on the permeation of active agent from the simplex formulation consisting of 1 % w/v solution of active alone in polar solvent.

**2. Study #2: Comparison of passive and cathodal iontophoretic delivery of active agent from simplex formulation**

Simplex formulation was prepared as 1% w/v active agent in water: ethanol (1:1) and it was investigated the effect of cathodal iontophoresis for 20 min on the delivery of active agent from the prepared simplex formulation into and through full thickness porcine skin. During this experiment, turbidity was observed in the donor solution (which was in contact with the silver chloride electrode) within 10 min of iontophoresis. The current was applied for 20 min and the amount of active in the receptor solution in the iontophoresis as well as corresponding passive test group was determined and the results have been shown in Fig 6. Figure 6 shows the amount of active in receptor solution.

No statistically significant difference was observed in the amount of active that permeated in passive as well as cathodal iontophoresis group ($p>0.05$) in this study. The standard deviation observed within each group was very high, and hence, a statistically insignificant difference was observed between the two test groups.

Also, the reason for appearance of turbidity in the donor solution was not clear; if it was due to precipitation of the active or it happened due to lack of sufficient ions in the formulation for conductivity. Therefore, only receptor samples were analyzed for this study and the complete experiment was run again in the next study with the change in the composition of the simplex formulation, to fix the issue of turbidity and confirm the observation of cathodal iontophoresis being ineffective in enhancing the delivery of the active compound.

**3. Study #3: Comparison of passive and cathodal iontophoretic delivery of active agent from modified simplex formulation**

In order to ensure the presence of sufficient conductive ions in the donor solution for the 20 min iontophoretic study, the simplex formulation used in study #2 was modified by preparing 1% w/v active agent solution in PBS (pH 7.4): ethanol (1:1). The results obtained have been shown in Fig 7. Figure 7 shows the amount of active in A. Receptor, B. Stratum corneum (tape strips) and C. Skin (viable epidermis and dermis).

Replacing water with buffer helped to counter the issue of turbidity observed in the donor.

However, no statistically significant difference ($p>0.05$) was observed between the two test groups (passive and iontophoresis) in terms of amount/sq.cm (ng/sq.cm) of the active quantified in the different compartments (receptor, stratum corneum (tapes), and skin).

This study, hence, confirmed the ineffectiveness of 20 min cathodal iontophoresis in enhancing the delivery of the active into and through full thickness porcine skin from the simplex formulation as well.

In the further studies, the effect of anodal iontophoresis on the skin permeation of active agent is investigated and compared with passive as well as cathodal iontophoresis.

**4. Study #4: Preliminary study to decide polarity for iontophoresis and modify the protocol for further investigations**

This study is performed to decide the polarity to be used for the iontophoretic delivery of active agent and modify the protocol for further investigations. Hence, this study investigated the effect of anodal iontophoresis on the delivery of active agent through dermatomed porcine skin, which was then compared with the cathodal iontophoretic as well as passive diffusion studies performed with similar conditions. Also, 1 % w/v solution of active agent in HEPES buffer (pH 7.15) containing 25 mM sodium chloride was used as the donor formulation in this study. This was done to investigate if the competitive ions from PBS used in the modified simplex formulation in study #3 were interfering with the delivery of active in case of cathodal iontophoresis. Constant current density of 0.2 mA/sq.cm was applied for 2 h and receptor sampling was performed at predetermined time points up to 4 h.

Overall, the purpose of investigating the formulation of active agent with different buffer as vehicle, using dermatomed porcine ear skin, applying current for longer duration using different polarities (anodal and cathodal for 2 h) as well as performing receptor analysis (for 4 h) was to get an idea as to which polarity would work better for the delivery of active agent and also if it was required to revisit the simplex formulation composition used in study # 3 and

then modify the protocol for further investigations. The results of this study have been shown in Figures 8a and 8b. Figure 8a shows the comparative permeation profile of the active agent over 4 h, and figure 8b shows the comparative delivery of the active after 20 min.

As shown in Fig.8a., application of anodal iontophoresis for 2 h was found to significantly enhance the permeation of the active agent through dermatomed porcine skin over 4 h study duration as compared to passive as well as cathodal iontophoresis ($p<0.05$). Moreover, the average cumulative amount of active delivered after 20 min was also significantly higher in the anodal iontophoresis group as compared to both passive as well as cathodal iontophoresis group ($p<0.05$) as shown in the bar graph in Fig. 8b. This, hence, showed that 20 min anodal iontophoresis was effective in enhancing the delivery of active agent. However, there was no significant difference in the cumulative active permeation between the passive and cathodal iontophoresis group ($p>0.05$) after 20 min or 4 h. This was in concordance with the results of studies 1-3 and it also confirmed that the presence of competitive ions in the modified simplex formulation (containing PBS) was not interfering with the delivery of the active upon application of cathodal iontophoresis.

### 5. Study #5: Investigation of 20 min anodal iontophoretic delivery of active agent from modified simplex formulation

This study was performed to investigate the effect of anodal iontophoresis (0.2 mA/sq.cm) applied for 20 min, on the delivery of active agent from modified simplex formulation, into and through full thickness porcine skin. The results of this investigation have been compared with those of passive and cathodal iontophoresis (study #3) and the comparative graphs of average amount/sq.cm (ng/sq.cm) ± SE of the active in receptor (amount permeated), tape strips (stratum corneum), and skin (viable epidermis and dermis) have been shown in Fig 9. Figure 9 shows the amount of active in A. Receptor, B. Stratum corneum (tape strips) and C. Skin (viable epidermis and dermis).

As shown in Fig 9A, the amount of the active that permeated from the solution into the receptor after 20 min was significantly higher in the anodal iontophoresis group as compared to passiveand cathodal iontophoresis test groups. Furthermore, the amount of active delivered into the viable part of the skin (epidermis and dermis) by anodal iontophoresis was also significantly higher than that delivered by passive diffusion as well as cathodal iontophoresis.

Therefore, these results showed that anodal iontophoresis was effective in enhancing the penetration of the active into the deeper viable layers of the skin, which was not observed in case of passive as well as cathodal iontophoresis groups, where most of the active was found in the stratum corneum. Thus, 20 min anodal iontophoresis successfully enhanced the permeation of the active agent from the modified simplex formulation, into and through the full thickness porcine skin.

### 6. Study #6: Investigation of 20 min anodal iontophoretic delivery of active agent from formulation FLUIDANOV™ + 1% active agent

This study was performed to investigate the effect of anodal iontophoresis (0.2 mA/sq.cm) applied for 20 min, on the delivery of active agent from formulation FLUIDANOV™ + 1 % active agent, into and through full thickness porcine skin. The results of this investigation have been compared with those of passive and cathodal iontophoresis (study #1) and the comparative graphs of average amount/sq.cm (ng/sq.cm) ± SE of active in receptor (amount permeated), tape strips (stratum corneum), and skin (viable epidermis and dermis) have been shown in Fig 10. Figure 10 shows the amount of active in A. Receptor, B. Stratum corneum (tape strips) and C. Skin (viable epidermis and dermis).

As shown in Fig 7A, the amount of the active that permeated from the formulation into the receptor after 20 min was significantly higher in the anodal iontophoresis group as compared to passive and cathodal iontophoresis test groups. Furthermore, the amount of active delivered into the viable epidermis and dermis by anodal iontophoresis was also significantly higher than that delivered by passive as well as cathodal. Also, the amount of active in the stratum corneum was found to be significantly higher in the anodal iontophoresis group as compared to passive control and cathodal iontophoresis group. Therefore, in case of formulation, in addition to the delivery of the active into deeper skin layers as well as receptor, anodal iontophoresis also enhanced the penetration of the active into stratum corneum as compared to passive and cathodal iontophoresis. Thus, 20 min anodal iontophoresis successfully enhanced the permeation of the active agent from formulation FLUIDANOV™ + 1 % active agent into and through the full thickness porcine skin.

### 7. Comparison of the delivery of the active agent from formulation FLUIDANOV™ + 1% active agent and modified simplex formulation into and through porcine skin using 20 min anodal iontophoresis

The results obtained from studies #5 and #6 have been compared and presented in Fig. 11. Overall, 20 min anodal iontophoretic treatment was found to enhance the delivery of the active agent, into and through full thickness porcine skin from both, the modified simplex formulation as well as formulation FLUIDANOV™ + 1% active agent.

However, the amount of active delivered into the receptor and viable skin, passively as well as by anodal iontophoresis from simplex formulation was greater than that delivered from formulation FLUIDANOV™ + 1 % active agent, which was expected as the complex composition as well as the viscous nature of the formulation would tend to reduce/retard the permeation of the active as compared to the simplex formulation (liquid).

Figure 11 shows a comparison of the amount of active delivered from the formulation FLUIDANOV™ + 1 % active agent and simplex formulation (solution) in A. Receptor and B. Viable layers of skin.

Anodal iontophoresis was found to enhance the delivery of the active agent from formulation FLUIDANOV™ + 1 % active agent as well as simplex formulation, into and through full thickness porcine ear skin. However, cathodal iontophoresis was not found to be effective for the same.

On the basis of structural aspects as well as pKa of 3.3, the active agent would be ionized at a pH > pKa (3.3). As the pH of simplex formulation was 4.07, the active would be negatively charged at this pH. Therefore, based on the charge, cathodal iontophoresis was expected to enhance delivery and was thus, investigated initially.

Anodal iontophoresis (20 min) was observed to enhance the delivery of the active agent from formulation FLUIDANOV™ + 1% active agent as well as the simplex formulation, into and through full thickness porcine ear skin.

While illustrative embodiments have been illustrated and described, it will be appreciated that various changes can be made therein without departing from the spirit and scope of the claimed subject matter.

## Claims

**1.** A iontophoresis method of delivering a N-acylaminoamide inhibitor of elastase through the skin, the electrical method comprising: applying a selected current profile, in particular a selected positive current profile, either continuous direct current, pulsed current or a combination of both, from any device and/or support comprising at least one electrode to a biological subject, the continuous direct current, the pulsed current or the combination of both of a character and for a duration sufficient to transdermally deliver a N-acylaminoamide inhibitor of elastase to a biological subject, and transporting different rates of N-acylaminoamide inhibitor of elastase across the skin in accordance to the selected current mode,
wherein the N-acylaminoamide inhibitor of elastase is a compound of formula (I):

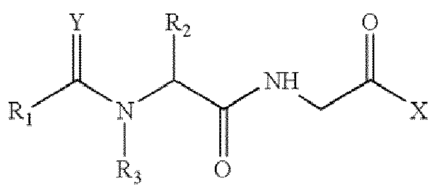

(I)

in which:

- the Y radical represents O or S;
- the $R_1$ radical represents:

(i) a hydrogen atom;
(ii) a hydrocarbon radical, linear, branched or cyclic, saturated or unsaturated, containing from 1 to 18 carbon atoms,
optionally substituted by 1 to 5 groups, identical or different, selected from the group consisting of -OH; -OR; -O-COR; -SH; -SR; -S-COR; $-NH_2$; -NHR; -NRR'; -NH-COR; -Hal (halogen); -CN; -COOR; -COR; $-P(O)-(OR)_2$; and $-SO_2-OR$;
with R and R' each representing independently a hydrocarbon radical, linear, branched or cyclic, saturated or unsaturated, containing 1 to 6 carbon atoms, optionally halogenated, said R and R' radicals being capable of forming together with N a 5- or 6- membered carbon ring that may include in addition at least one heteroatom selected from the group consisting of O, N and/or S in the ring, and/or may be substituted by 1 to 5 groups, identical or different, selected from the group consisting of -OH; -OR"; -O-COR"; -SH; -SR"; -S-COR"; $-NH_2$; -NHR"; -NH-COR"; -Hal (halogen); -CN; -COOR"; -COR"; $-C-Hal_3$ (halogen); with R" representing independently a hydrocarbon radical, linear, branched or cyclic, saturated or unsaturated, containing 1 to 6 carbon atoms, optionally halogenated;
(iii) a radical selected from the group consisting of the radicals -OR; $-NH_2$; -NHR; -NRR'; -NH-COR; -COOR; -COR;

with R and R' each representing independently a hydrocarbon radical, linear, branched or cyclic, saturated or unsaturated, containing 1 to 6 carbon atoms, optionally halogenated; said R and R' radicals being capable of forming together with N a 5- or 6- membered carbon ring that may include in addition at least one heteroatom selected from the group consisting of O, N and/or S in the ring, and/or may be substituted by 1 to 5 groups, identical or different, selected from the group consisting of -OH; -OR"; -O-COR"; -SH; -SR"; -S-COR"; $-NH_2$; -NHR"; -NH-COR"; -Hal (halogen); -CN; -COOR"; and -COR"; with R" representing a hydrocarbon radical, linear, branched or cyclic, saturated or unsaturated, containing 1 to 6 carbon atoms, optionally halogenated,

- the radical $R_2$ represents a hydrocarbon radical, linear, branched or cyclic saturated or unsaturated, containing 1 to 18 carbon atoms, optionally substituted by 1 to 5 groups, identical or different, selected from the group consisting of -OH; -OR; -O-COR; -SH; -SR; -S-COR; $-NH_2$; -NHR; -NRR"; -NH-COR; -Hal (halogen); -CN; -COOR; and -COR;
with R and R' each representing independently a hydrocarbon radical, linear, branched or cyclic, saturated or unsaturated, containing 1 to 6 carbon atoms, optionally halogenated, said R and R' radicals being capable of forming together with N a 5- or 6- membered carbon ring that may include in addition at least one heteroatom selected from the group consisting of O, N and/or S in the ring, and/or may be substituted by 1 to 5 groups, identical or different, selected from the group consisting of -OH; -OR"; -O-COR"; -SH; -SR"; -S-COR"; $-NH_2$; -NHR"; -NH-COR"; -Hal (halogen); -CN; -COOR"; and -COR"; with R" representing a hydrocarbon radical, linear, branched or cyclic, saturated or unsaturated, containing 1 to 6 carbon atoms, optionally halogenated,
- the radical $R_3$ represents a radical selected from the group consisting of those of formula (II) or (III) :

$$-A-C_6H_{(5-y)}-B_y \qquad (II)$$

$$-C_6H_{(5-y)}-B_{y'} \qquad (III)$$

in which:

- y is an integer included between 0 and 5 ;
- y' is an integer included between 1 and 5;
- A is a divalent hydrocarbon radical, linear, branched or cyclic, saturated or unsaturated, containing 1 to 18 carbon atoms, optionally substituted by 1 to 5 groups, identical or different, selected from the group consisting of -OH; -OR; -O-COR; -SH; -SR; -S-COR; $-NH_2$; -NHR; -NRR'; -NH-COR; -Hal (halogen); -CN; -COOR; -COR; $-NO_2$; and $-SO_2$-OR;
with R and R' each representing independently a hydrocarbon radical, linear, branched or cyclic, saturated or unsaturated, containing 1 to 6 carbon atoms, optionally halogenated, said R and R' radicals being capable of forming together with N a 5- or 6- membered carbon ring that may include in addition at least one heteroatom selected from the group consisting of O, N and/or S in the ring, and/or may be substituted by 1 to 5 groups, identical or different, selected from the group consisting of -OH; -OR"; -O-COR"; -SH; -SR"; -S-COR"; $-NH_2$; -NHR"; -NH-COR"; -Hal (halogen); -CN; -COOR"; and -COR"; with R" representing a hydrocarbon radical, linear, branched or cyclic, saturated or unsaturated, containing 1 to 6 carbon atoms, optionally halogenated ;
- B is a hydrocarbon radical, linear, branched or cyclic, saturated or unsaturated, containing 1 to 18 carbon atoms, optionally substituted by 1 to 5 groups, identical or different, selected from the group consisting of -OH; -OR; -O-COR; -SH; -SR; -S-COR; $-NH_2$; -NHR; -NRR'; -NH-COR; -Hal (halogen); $-C-Hal_3$ (halogen); -CN; -COOR; -COR; $-NO_2$; and $-SO_2$-OR;
with R and R' each representing independently a hydrocarbon radical, linear, branched or cyclic, saturated or unsaturated; containing 1 to 6 carbon atoms, optionally halogenated, said R and R' radicals being capable of forming together with N a 5- or 6- membered carbon ring that may include in addition at least one heteroatom selected from the group consisting of O, N and/or S in the ring, and/or may be substituted by 1 to 5 groups, identical or different, selected from the group consisting of -OH; -OR"; -O-COR"; -SH; -SR"; -S-COR"; $-NH_2$; -NHR"; -NH-COR"; -Hal (halogen); -CN; -COOR"; and -COR"; with R" representing a hydrocarbon radical, linear, branched or cyclic, saturated or unsaturated, containing 1 to 6 carbon atoms, optionally halogenated ;

- the radical X represents a radical selected from the group consisting of -OH; $-OR_4$, $-NH_2$, $-NHR_4$, $-NR_4R_5$, $-SR_4$, $-COOR_4$; and $-COR_4$;

with $R_4$ and $R_5$ each representing independently a hydrocarbon radical, linear, branched or cyclic, saturated or unsaturated, containing 1 to 6 carbon atoms, optionally substituted by 1 to 5 groups, identical or different, selected from the group consisting of -OH; -OR; -O-COR; -SH; -SR; -S-COR; $-NH_2$; -NHR; -NRR'; -NH-COR; -Hal (halogen); -CN; -COOR; and -COR;
with R and R' each representing independently a hydrocarbon radical, linear, branched or cyclic, saturated or unsaturated, containing 1 to 6 carbon atoms, optionally halogenated, said R and R' radicals being capable of forming together with N a 5- or 6- membered carbon ring that may include in addition at least one heteroatom selected from the group consisting of O, N and/or S in the ring, and/or may be substituted by 1 to 5 groups, identical or different, selected from the group consisting of -OH; -OR"; -O-COR"; -SH; -SR"; -S-COR"; $-NH_2$; -NHR"; -NH-COR"; -Hal (halogen); -CN; -COOR"; and -COR"; with R" representing a hydrocarbon radical, linear, branched or cyclic, saturated or unsaturated, containing 1 to 6 carbon atoms, optionally halogenated,
said $R_4$ and $R_5$ radicals being capable of forming together with N a 5- or 6 membered carbon ring that may include in addition at least one heteroatom selected from the group consisting of O, N and/or S in the ring, and/or may be substituted by 1 to 5 groups, identical or different, selected from the group consisting of -OH; -OR"; -O-COR"; -SH; -SR"; -S-COR"; $-NH_2$; -NHR"; -NH-COR"; -Hal (halogen); -CN; -COOR"; -COR"; with R" representing a hydrocarbon radical, linear, branched or cyclic, saturated or unsaturated, containing 1 to 6 carbon atoms, optionally halogenated, its mineral or organic acid salts, its optical isomers, in isolated form or as a racemic mixture.

**2.** The iontophoresis method of the preceding claim, wherein the Y radical represents O and/or the X radical represents -OH.

**3.** The iontophoresis method of any preceding claim, wherein applying a selected current profile to a biological subject includes generating a continuous direct current stimulus, in particular a continuous positive direct current stimulus, having an average current density ranging from 0.001 $mA/cm^2$ to 0.5 $mA/cm^2$, preferably from 0.005 $mA/cm^2$ to 0.4 $mA/cm^2$, preferably from 0.01 $mA/cm^2$ to 0.3 $mA/cm^2$, and more preferably from 0.05 $mA/cm^2$ to 0.2 $mA/cm^2$, and in particular of 0.2 $mA/cm^2$.

**4.** The iontophoresis method of the preceding claim, wherein the continuous direct current stimulus, in particular a continuous positive direct current stimulus, is applied for a duration ranging from 1 minute to 60 minutes, preferably from 5 minutes to 50 minutes, and more preferably from 10 minute to 40 minutes.

**5.** The iontophoresis method of any preceding claim, further comprising:

transdermally delivering a composition including one or more of N-acetyl-3-trifluoromethyl-phenyl-(valine-glycine), N-acetyl-3-trifluoromethyl-phenyl-(valine-glycine) derivatives, ions of N-acetyl-3-trifluoromethyl-phenyl-(valine-glycine), and ions of N-acetyl-3-trifluoromethyl-phenyl-(valine-glycine) derivatives.

**6.** The iontophoresis method of any preceding claim, further comprising:

transdermally delivering a composition comprising the N-acylaminoamide inhibitor of elastase present in amounts ranging from 0.01 % to 100 % by weight, preferably from 0.5 % to 60 % by weight.

**7.** The iontophoresis method of any preceding claim, further comprising:

transdermally delivering a composition including N-acylaminoamide inhibitor of elastase, and in particular one or more of N-acetyl-3-trifluoromethyl-phenyl-(valine-glycine), N-acetyl-3-trifluoromethyl-phenyl-(valine-glycine) derivatives, ions of N-acetyl-3-trifluoromethyl-phenyl-(valine-glycine), and ions of N-acetyl-3-trifluoromethyl-phenyl-(valine-glycine) derivatives, present in an amount of 1 % by weight.

**8.** The iontophoresis method according to any preceding claims, comprising the step of measuring at least one of the temperatures of the skin, the impedance of the skin, and a pH of the composition.

**9.** The iontophoresis method according to the preceding claims, wherein the application of current profile may be reduced to a safety level when a measured value measured by one of the sensors exceeds a safety range or a safety value.

**10.** A iontophoresis composition, comprising:

N-acylaminoamide inhibitor of elastase, and in particular one or more of N-acetyl-3-trifluoromethyl-phenyl-(valine-glycine), N-acetyl-3-trifluoromethyl-phenyl-(valine-glycine) derivatives, ions of N-acetyl-3-trifluoromethyl-phenyl-(valine-glycine), and ions of N-acetyl-3-trifluoromethyl-phenyl-(valine-glycine) derivatives, present in amounts ranging from 0.1 % to 30 % by weight, preferably in an amounts ranging from 0.5 % to 20 % by weight, 0.7 % to 10 % by weight, and
water present in an amount of at least 20 % by weight, preferably in an amount of at least 40 % by weight, more preferably in an amount of at least 45 % by weight.

**11.** The iontophoresis composition according to the preceding claim, also comprising: ethanol present in an amount of at least 20 % by weight, preferably in an amount of at least 40 % by weight, more preferably in an amount of at least 45 % by weight.

**12.** The iontophoresis composition according to claim 10 or 11, in the form of an emulsion, in particular in the form of an oil-in-water emulsion, preferably containing a surfactant, and more preferably containing a silicon surfactant.

**13.** A iontophoresis kit comprising:

- A iontophoresis composition including N-acylaminoamide inhibitor of elastase, and in particular one or more of N-acetyl-3-trifluoromethyl-phenyl-(valine-glycine), N-acetyl-3-trifluoromethyl-phenyl-(valine-glycine) derivatives, ions of N-acetyl-3-trifluoromethyl-phenyl-(valine-glycine), and ions of N-acetyl-3-trifluoromethyl-phenyl-(valine-glycine) derivatives, and
- An iontophoresis device for carrying the iontophoresis method of anyone of claims 1 to 9.

**14.** A iontophoresis kit comprising:

- A iontophoresis composition including N-acylaminoamide inhibitor of elastase, and in particular one or more of N-acetyl-3-trifluoromethyl-phenyl-(valine-glycine), N-acetyl-3-trifluoromethyl-phenyl-(valine-glycine) derivatives, ions of N-acetyl-3-trifluoromethyl-phenyl-(valine-glycine), and ions of N-acetyl-3-trifluoromethyl-phenyl-(valine-glycine) derivatives, and
- An iontophoresis device for delivering the iontophoresis composition through the skin, configured for applying a selected current profile, in particular a continuous positive direct current stimulus, either continuous direct current, pulsed current or a combination of both.

**15.** The iontophoresis kit of the two preceding claims, wherein the composition is an aqueous composition.

**16.** The iontophoresis kit of any one of the three preceding claims, the kit being configured such that N-acylaminoamide inhibitor of elastase, and in particular N-acetyl-3-trifluoromethyl-phenyl-(valine-glycine), and water are already mixed in the composition when the composition is applied to the skin.

**17.** The iontophoresis kit of any one of claims 14 to 16, the device comprising at least one of a temperature sensor, an impedance sensor, and a pH sensor, wherein the device may be configured such that the application of current profile is reduced to a safety level when a measured value measured by one of the sensors exceeds a safety range or a safety value.

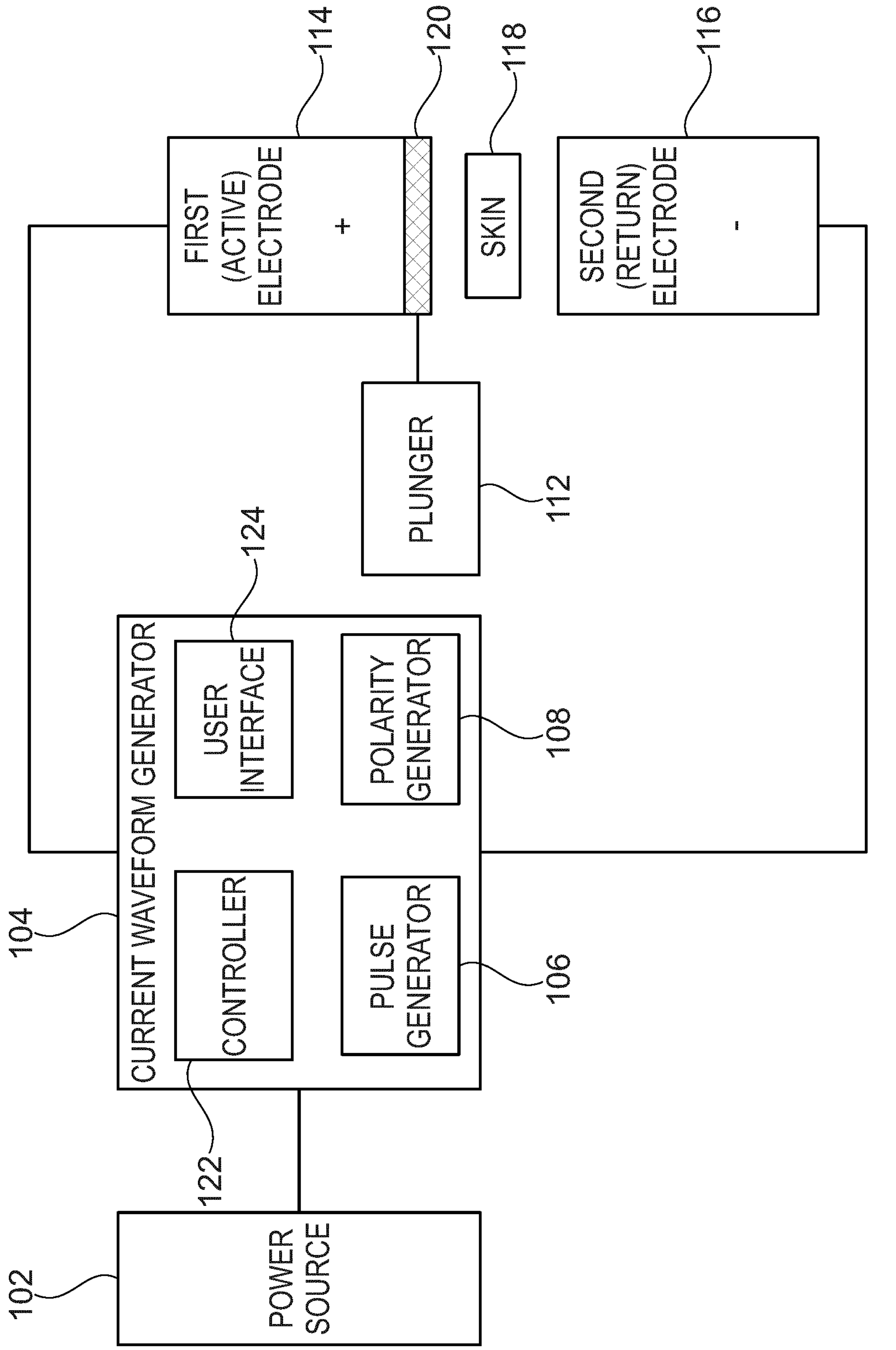
114
120
118
116
FIRST
(ACTIVE)
ELECTRODE
+
SKIN
SECOND
(RETURN)
ELECTRODE
-
PLUNGER
112
124
CURRENT WAVEFORM GENERATOR
USER
INTERFACE
POLARITY
GENERATOR
108
CONTROLLER
PULSE
GENERATOR
106
104
122
POWER
SOURCE
102

Fig. 1

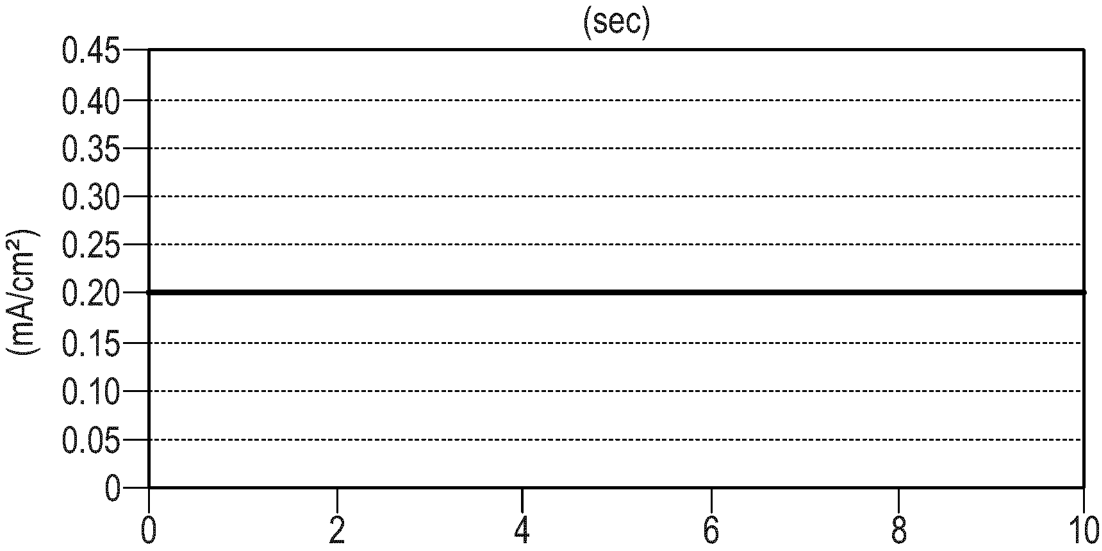
(sec)
(mA/cm²)
0.45
0.40
0.35
0.30
0.25
0.20
0.15
0.10
0.05
0
0
2
4
6
8
10

Fig. 2

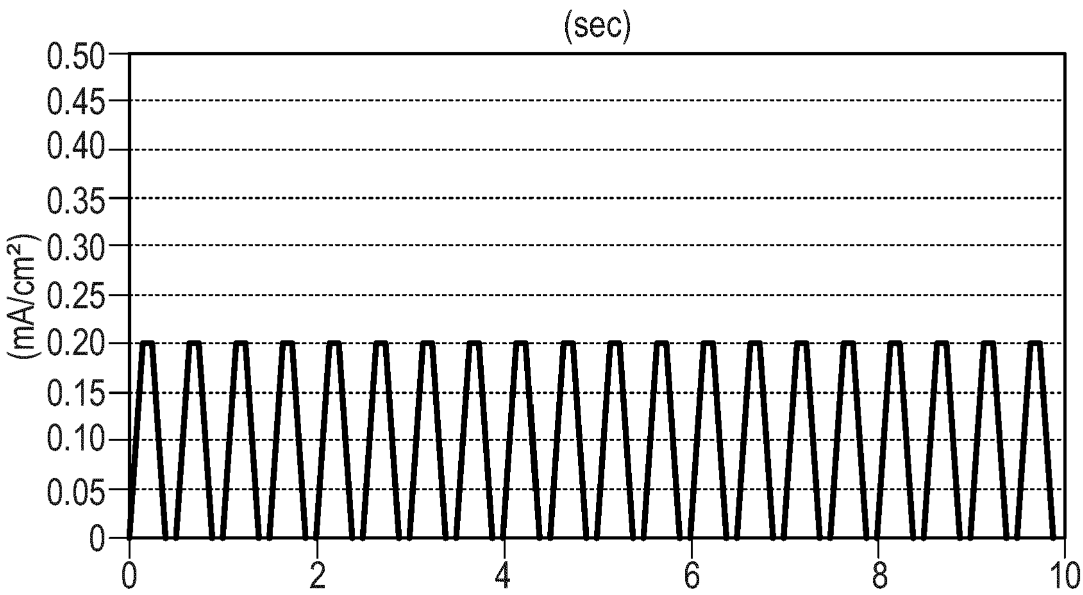
(sec)
(mA/cm²)
0.50
0.45
0.40
0.35
0.30
0.25
0.20
0.15
0.10
0.05
0
0
2
4
6
8
10

Fig. 3

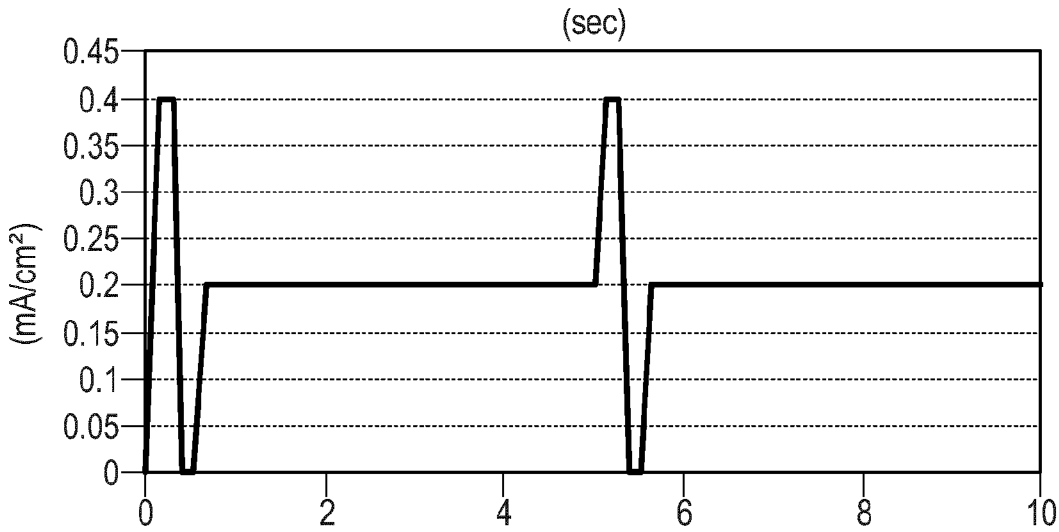
(sec)
(mA/cm²)
0.45
0.4
0.35
0.3
0.25
0.2
0.15
0.1
0.05
0
0
2
4
6
8
10

Fig. 4

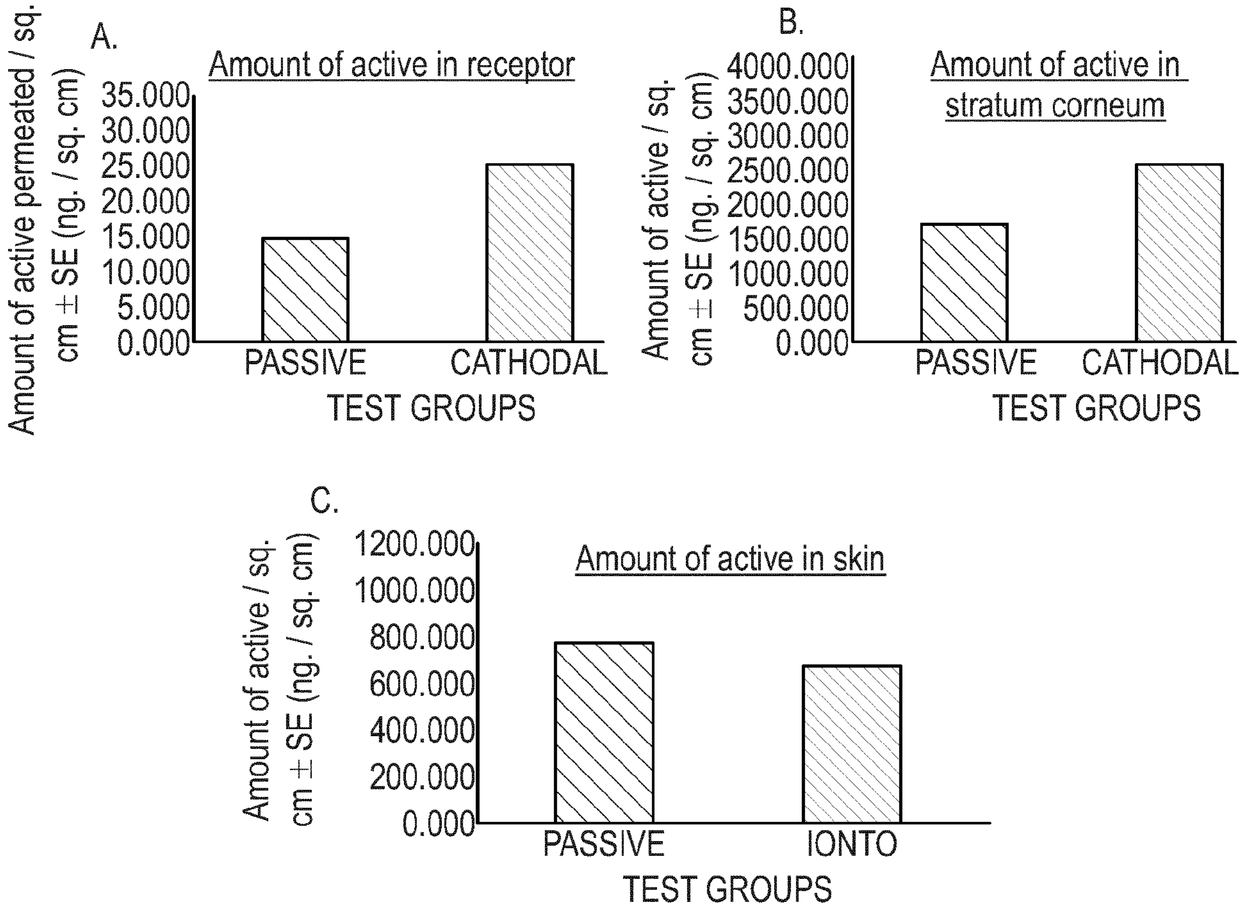
A.
Amount of active in receptor
Amount of active permeated / sq. cm ± SE (ng. / sq. cm)
35.000
30.000
25.000
20.000
15.000
10.000
5.000
0.000
PASSIVE
CATHODAL
TEST GROUPS
B.
Amount of active in stratum corneum
Amount of active / sq. cm ± SE (ng. / sq. cm)
4000.000
3500.000
3000.000
2500.000
2000.000
1500.000
1000.000
500.000
0.000
PASSIVE
CATHODAL
TEST GROUPS
C.
Amount of active in skin
Amount of active / sq. cm ± SE (ng. / sq. cm)
1200.000
1000.000
800.000
600.000
400.000
200.000
0.000
PASSIVE
IONTO
TEST GROUPS

Fig. 5

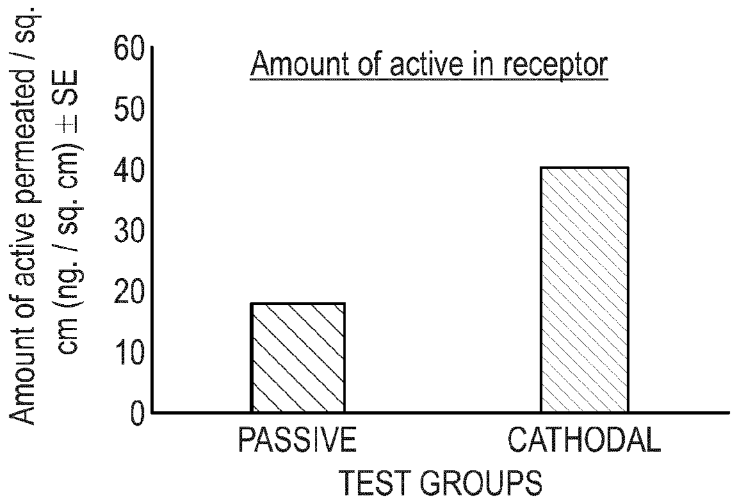
Amount of active in receptor
Amount of active permeated / sq. cm (ng. / sq. cm) ± SE
60
50
40
30
20
10
0
PASSIVE
CATHODAL
TEST GROUPS

Fig. 6

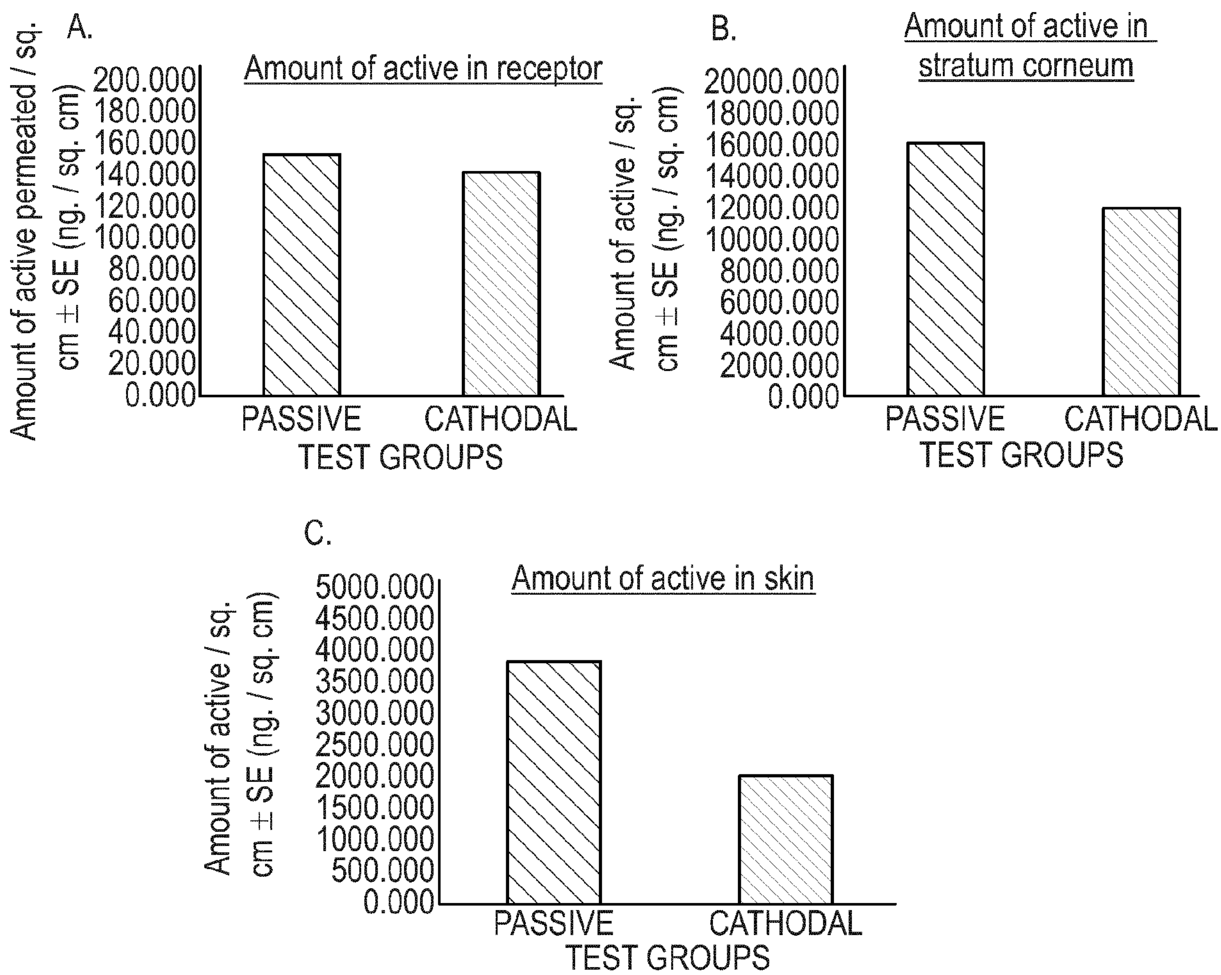
A.
Amount of active in receptor
Amount of active permeated / sq. cm ± SE (ng. / sq. cm)
200.000
180.000
160.000
140.000
120.000
100.000
80.000
60.000
40.000
20.000
0.000
PASSIVE
CATHODAL
TEST GROUPS
B.
Amount of active in stratum corneum
Amount of active / sq. cm ± SE (ng. / sq. cm)
20000.000
18000.000
16000.000
14000.000
12000.000
10000.000
8000.000
6000.000
4000.000
2000.000
0.000
PASSIVE
CATHODAL
TEST GROUPS
C.
Amount of active in skin
Amount of active / sq. cm ± SE (ng. / sq. cm)
5000.000
4500.000
4000.000
3500.000
3000.000
2500.000
2000.000
1500.000
1000.000
500.000
0.000
PASSIVE
CATHODAL
TEST GROUPS

Fig. 7

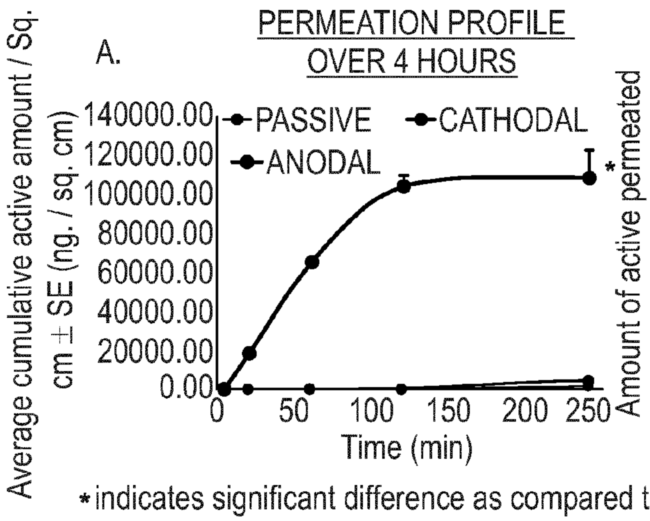


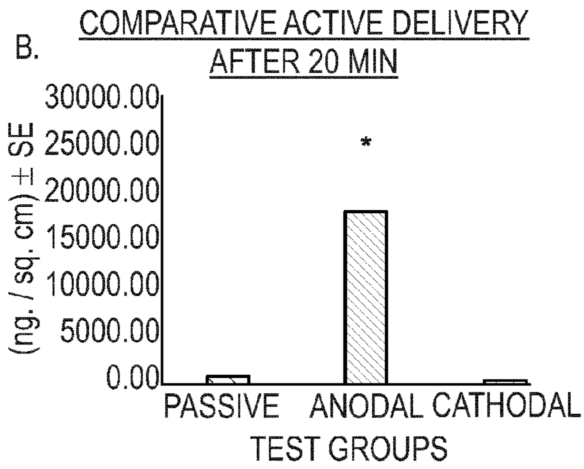


*indicates significant difference as compared to the other test groups ($p < 0.05$)

Fig. 8a

Fig. 8b

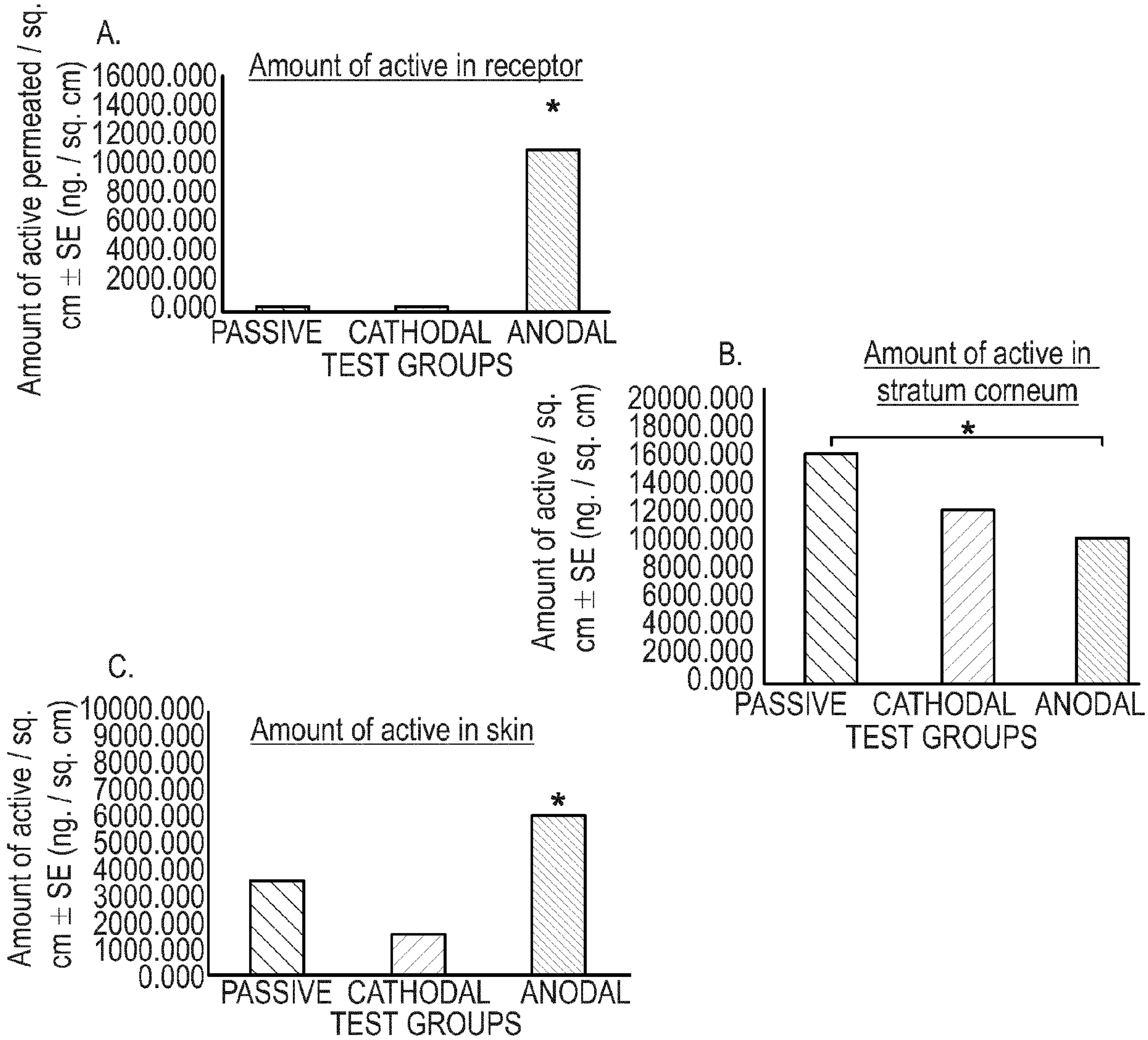


Fig. 9

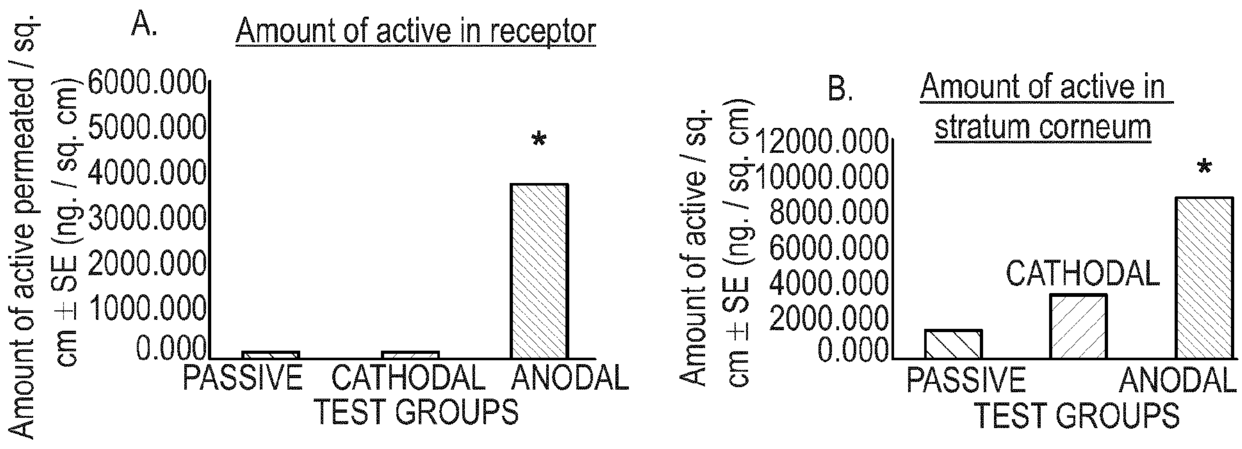
A.
Amount of active in receptor
Amount of active permeated / sq. cm ± SE (ng. / sq. cm)
6000.000
5000.000
4000.000
3000.000
2000.000
1000.000
0.000
*
PASSIVE
CATHODAL
ANODAL
TEST GROUPS
B.
Amount of active in stratum corneum
Amount of active / sq. cm ± SE (ng. / sq. cm)
12000.000
10000.000
8000.000
6000.000
4000.000
2000.000
0.000
*
CATHODAL
PASSIVE
ANODAL
TEST GROUPS
C.
Amount of active in skin
Amount of active / sq. cm ± SE (ng. / sq. cm)
6000.000
5000.000
4000.000
3000.000
2000.000
1000.000
0.000
*
PASSIVE
CATHODAL
ANODAL
TEST GROUPS

Fig. 10

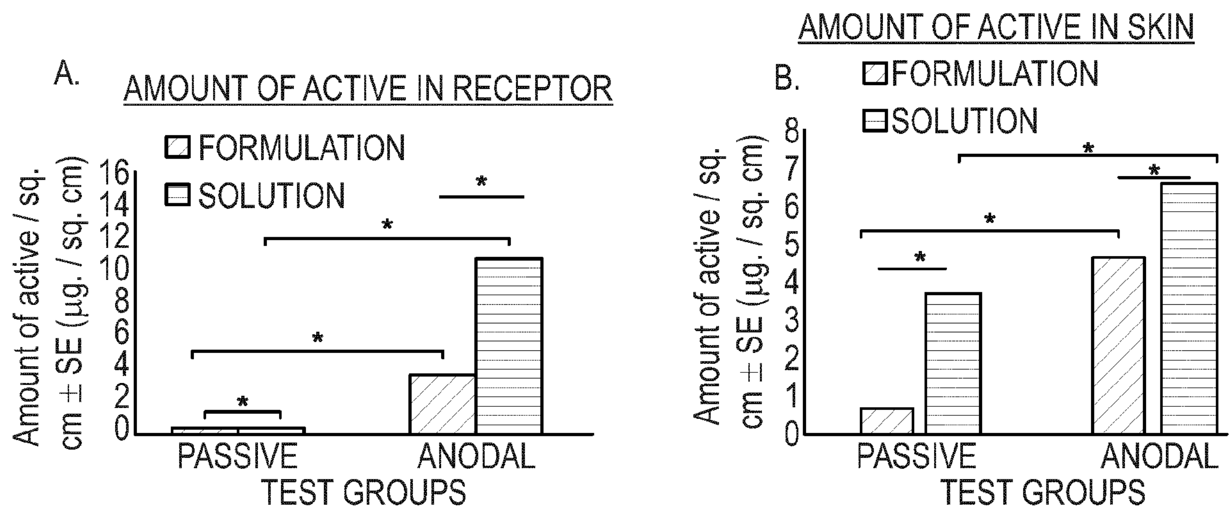
A.
AMOUNT OF ACTIVE IN RECEPTOR
FORMULATION
SOLUTION
Amount of active / sq. cm ± SE (µg. / sq. cm)
16
14
12
10
8
6
4
2
0
*
PASSIVE
ANODAL
TEST GROUPS
AMOUNT OF ACTIVE IN SKIN
B.
FORMULATION
SOLUTION
Amount of active / sq. cm ± SE (µg. / sq. cm)
8
7
6
5
4
3
2
1
0
*
PASSIVE
ANODAL
TEST GROUPS

Fig. 11

Europäisches Patentamt
European Patent Office
Office européen des brevets

# EUROPEAN SEARCH REPORT

Application Number
EP 17 30 6000

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2010/091893 A1 (LIPOTEC SA [ES]; GARCIA SANZ NURIA [ES]; VAN DEN NEST WIM [ES]; CARREN) 19 August 2010 (2010-08-19)<br>* page 1, line 5 - line 11 *<br>* page 26, line 20 - line 23 *<br>* page 27, line 25 - line 30 *<br>* page 38, line 21 ff. *<br>----- | 1-9, 13-17 | INV.<br>A61K8/64<br>A61Q19/08 |
| Y | US 2011/021438 A1 (DALKO MARIA [FR] ET AL) 27 January 2011 (2011-01-27)<br>* paragraphs [0118], [0127], [0134] *<br>----- | 1-9, 13-17 | |
| Y | WO 2016/096804 A1 (OREAL [FR]) 23 June 2016 (2016-06-23)<br>* claims 1,15 *<br>----- | 1-9, 13-17 | |
| | | | TECHNICAL FIELDS SEARCHED (IPC) |
| | | | A61K<br>A61Q |

~~The present search report has been drawn up for all claims~~

1

| Place of search | Date of completion of the search | Examiner |
|---|---|---|
| Munich | 11 October 2017 | Lenzen, Achim |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number
EP 17 30 6000

## CLAIMS INCURRING FEES

The present European patent application comprised at the time of filing claims for which payment was due.

- [ ] Only part of the claims have been paid within the prescribed time limit. The present European search report has been drawn up for those claims for which no payment was due and for those claims for which claims fees have been paid, namely claim(s):

- [ ] No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

- [ ] All further search fees have been paid within the fixed time limit. The present European search report has been drawn up for all claims.

- [ ] As all searchable claims could be searched without effort justifying an additional fee, the Search Division did not invite payment of any additional fee.

- [ ] Only part of the further search fees have been paid within the fixed time limit. The present European search report has been drawn up for those parts of the European patent application which relate to the inventions in respect of which search fees have been paid, namely claims:

- [X] None of the further search fees have been paid within the fixed time limit. The present European search report has been drawn up for those parts of the European patent application which relate to the invention first mentioned in the claims, namely claims:

  1-9, 13-17

- [ ] The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION
# SHEET B

Application Number
EP 17 30 6000

The Search Division considers that the present European patent application does not comply with the requirements of unity of invention and relates to several inventions or groups of inventions, namely:

1. claims: 1-9, 13-17

A iontophoresis method employing the compounds of formula (I) (claims 1-9), iontophoresis kits comprising a composition containing N-acetyl-3-trifluoromethyl-phenyl-(valine-glycine) (ions/derivatives thereof) and a iontophoresis device (claims 13-17)

---

2. claims: 10-12

Composition comprising a N-acylaminoamide inhibitor of elastase

---

## ANNEX TO THE EUROPEAN SEARCH REPORT ON EUROPEAN PATENT APPLICATION NO. EP 17 30 6000

This annex lists the patent family members relating to the patent documents cited in the above-mentioned European search report. The members are as contained in the European Patent Office EDP file on
The European Patent Office is in no way liable for these particulars which are merely given for the purpose of information.

11-10-2017

| Patent document cited in search report | Publication date | Patent family member(s) | Publication date |
|---|---|---|---|
| WO 2010091893 A1 | 19-08-2010 | AU 2010213094 A1 | 18-08-2011 |
| | | CA 2751436 A1 | 19-08-2010 |
| | | CN 102317307 A | 11-01-2012 |
| | | EP 2408801 A1 | 25-01-2012 |
| | | ES 2349972 A1 | 13-01-2011 |
| | | ES 2547762 T3 | 08-10-2015 |
| | | HK 1162540 A1 | 29-04-2016 |
| | | JP 5878761 B2 | 08-03-2016 |
| | | JP 2012519656 A | 30-08-2012 |
| | | KR 20110125247 A | 18-11-2011 |
| | | US 2011300199 A1 | 08-12-2011 |
| | | WO 2010091893 A1 | 19-08-2010 |
| US 2011021438 A1 | 27-01-2011 | AR 028678 A1 | 21-05-2003 |
| | | AT 271562 T | 15-08-2004 |
| | | AU 6247501 A | 17-12-2001 |
| | | BR 0111648 A | 01-07-2003 |
| | | CA 2408670 A1 | 13-12-2001 |
| | | CN 1436197 A | 13-08-2003 |
| | | DE 60104423 D1 | 26-08-2004 |
| | | DE 60104423 T2 | 25-08-2005 |
| | | EP 1292608 A2 | 19-03-2003 |
| | | ES 2223862 T3 | 01-03-2005 |
| | | FR 2810033 A1 | 14-12-2001 |
| | | HU 0302744 A2 | 28-11-2003 |
| | | JP 3843943 B2 | 08-11-2006 |
| | | JP 2003535872 A | 02-12-2003 |
| | | KR 20030022138 A | 15-03-2003 |
| | | MX PA02012000 A | 22-04-2003 |
| | | PL 359064 A1 | 23-08-2004 |
| | | US 2003152600 A1 | 14-08-2003 |
| | | US 2005187197 A1 | 25-08-2005 |
| | | US 2008293962 A1 | 27-11-2008 |
| | | US 2011021438 A1 | 27-01-2011 |
| | | WO 0194381 A2 | 13-12-2001 |
| WO 2016096804 A1 | 23-06-2016 | CN 107001412 A | 01-08-2017 |
| | | EP 3233886 A1 | 25-10-2017 |
| | | FR 3030523 A1 | 24-06-2016 |
| | | JP 2018501239 A | 18-01-2018 |
| | | KR 20170088420 A | 01-08-2017 |
| | | US 2017360667 A1 | 21-12-2017 |
| | | WO 2016096804 A1 | 23-06-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2408801 A
- WO 2010118880 A


### Non-patent literature cited in the description


- **SANTI, P. ; R. H. GUY.** Reverse iontophoresis-parameters determining electroosmotic flow: I. pH and ionic strength. *J. Control. Release,* 1996, vol. 38, 159-165
- **SANTI, P. ; R. H. GUY.** Reverse iontophoresis-parameters determining electroosmotic flow: I. pH and ionic strength. *J. Control. Release.,* 1996, vol. 38, 159-165
- **KIRK-OTHMER'S.** Encyclopedia of Chemical Technology. Wiley, 1979, vol. 22, 333-432